# FASCICULE DE BREVET EUROPEEN

(11) **EP 2 768 842 B1**
(45) Date de publication et mention de la délivrance du brevet: **25.07.2018**
(21) Numéro de dépôt: 12772951.5
(22) Date de dépôt: 16.10.2012
(51) Int. Cl.: C07K 1/04, C07K 1/02

(54) **PROCÉDÉ DE SYNTHÈSE DE PROTÉINES**
VERFAHREN ZUR SYNTHESE VON PROTEINEN
METHOD FOR SYNTHESIZING PROTEINS

(30) Priorité: 17.10.2011 FR 1159348
(43) Date de publication de la demande: 27.08.2014
(73) Titulaire: Centre National de la Recherche Scientifique (C.N.R.S.), 75794 Paris Cedex 16 (FR)
(72) Inventeur: MELNYK, Oleg, Annoeullin 59112 (FR); RAIBAUT, Laurent, 06000 Nice (FR); AUCAGNE, Vincent, 45400 Fleury-les-Aubrais (FR); DELMAS, Agnès, 45000 Orleans (FR)
(74) Mandataire: Lavoix
(86) Numéro de dépôt international: PCT/EP2012/070454
(87) Numéro de publication internationale: WO 2013/057084

(56) Documents cités:
- FR-A1- 2 952 058
- US-A1- 2002 132 975

### SEQUENCE LISTING

<110> CNRS
<120> Procédé de synthèse de protéines
<130> 33259 FR
<160> 9
<170> PatentIn version 3.5
<210> 1
   <211> 10
   <212> PRT
   <213> Artificial Sequence
<220>
   <223> Peptide X1
<400> 1
   Gly Ile Leu Lys Glu Pro Val Gln Gly Ala
   1               5                   10
<210> 2
   <211> 10
   <212> PRT
   <213> Artificial Sequence
<220>
   <223> Peptide 9
<400> 2
   Cys Ile Leu Lys Glu Pro Val His Gly Ala
   1               5                   10
<210> 3
   <211> 36
   <212> PRT
   <213> Artificial Sequence
<220>
   <223> Peptide 11a
<400> 3
   Gly Ile Leu Lys Glu Pro Val Gln Gly Ala Cys His His Leu Glu Pro
   1               5                   10                  15
   Gly Gly Cys His His Leu Glu Pro Ala Gly Cys Ile Leu Lys Glu Pro
               20                  25                  30
   Val His Gly Ala
           35
<210> 4
   <211> 18
   <212> PRT
   <213> Artificial Sequence
<220>
   <223> Peptide 16a
<400> 4
   Cys His His Leu Glu Pro Gly Gly Cys Ile Leu Lys Glu Pro Val His
   1               5                   10                  15
   Gly Ala
<210> 5
   <211> 94
   <212> PRT
   <213> Artificial Sequence
<220>
   <223> Peptide 29
<220>
   <221> VARIANT
   <222> (94)..(94)
   <223> Xaa is Lys biotinylated
<400> 5
   Ala Ala Ala Ala Ala Lys Asp Tyr Ile Arg Asn Cys Ile Ile Gly Lys
   1               5                   10                  15
   Gly Arg Ser Tyr Lys Gly Thr Val Ser Ile Thr Lys Ser Gly Ile Lys
               20                  25                  30
   Cys Gln Pro Trp Ser Ser Met Ile Pro His Glu His Ser Phe Leu Pro
           35                  40                  45
   Ser Ser Tyr Arg Gly Lys Asp Leu Gln Glu Asn Tyr Cys Arg Asn Pro
       50                  55                  60
   Arg Gly Glu Glu Gly Gly Pro Trp Cys Phe Thr Ser Asn Pro Glu Val
   65                  70                  75                  80
   Arg Tyr Glu Val Cys Asp Ile Pro Gln Cys Ser Glu Val Xaa
                   85                  90
<210> 6
   <211> 11
   <212> PRT
   <213> Artificial Sequence
<220>
   <223> Peptide 21
<220>
   <221> VARIANT
   <222> (11)..(11)
   <223> Xaa is Asn-SEAoff in which SEAoff means bis(2-sulfanylethyl)amino cyclic
<400> 6
   Ala Ala Ala Ala Ala Lys Asp Tyr Ile Arg Xaa
   1               5                   10
<210> 7
   <211> 21
   <212> PRT
   <213> Artificial Sequence
<220>
   <223> Peptide 22
<220>
   <221> VARIANT
   <222> (1)..(1)
   <223> Xaa at position 1 is Cys(StBu) in which StBu means terbutylsulfenyl
<220>
   <221> VARIANT
   <222> (21)..(21)
   <223> Xaa at position 21 is Lys-SEAoff, in which SEAoff means bis(2-sulfanylethyl)amino cyclic
<400> 7
   Xaa Ile Ile Gly Lys Gly Arg Ser Tyr Lys Gly Thr Val Ser Ile Thr
   1               5                   10                  15
   Lys Ser Gly Ile Xaa
               20
<210> 8
   <211> 28
   <212> PRT
   <213> Artificial Sequence
<220>
   <223> Peptide 25
<220>
   <221> VARIANT
   <222> (1)..(1)
   <223> Xaa at position 1 is Cys(StBu) in which StBu means terbutylsulfenyl
<220>
   <221> VARIANT
   <222> (28)..(28)
   <223> Xaa at position 28 is Tyr-SEAoff in which SEAoff means bis(2-sulfanylethyl)amino cyclic
<400> 8
   Xaa Gln Pro Trp Ser Ser Met Ile Pro His Glu His Ser Phe Leu Pro
   1               5                   10                  15
   Ser Ser Tyr Arg Gly Lys Asp Leu Gln Glu Asn Xaa
               20                  25
<210> 9
   <211> 34
   <212> PRT
   <213> Artificial Sequence
<220>
   <223> Peptide 27
<220>
   <221> VARIANT
   <222> (34)..(34)
   <223> Xaa is Lys biotinylated
<400> 9
   Cys Arg Asn Pro Arg Gly Glu Glu Gly Gly Pro Trp Cys Phe Thr Ser
   1               5                   10                  15
   Asn Pro Glu Val Arg Tyr Glu Val Cys Asp Ile Pro Gln Cys Ser Glu
               20                  25                  30
   Val Xaa

## Description

### DOMAINE DE L'INVENTION

La présente invention concerne un nouveau procédé d'assemblage de protéines à partir de fragments peptidiques. Il permet la production de protéines de façon simple, fiable, automatisable et applicable à l'échelle industrielle. Ce procédé permet la production de protéines d'intérêt thérapeutique ou diagnostique. L'invention a également pour objet des kits et des dispositifs automatisés permettant de mettre en oeuvre ce procédé de synthèse ainsi que des kits de test et/ou de diagnostic.

### ARRIERE-PLAN TECHNOLOGIQUE

La synthèse de polypeptides par des méthodes conventionnelles en phase solide, acide aminé par acide aminé, est limitée par des rendements faibles lorsque les polypeptides synthétisés sont de grande taille. Afin de surmonter cette limitation, il est connu d'assembler deux polypeptides par ligation chimique afin de produire un polypeptide plus long.

La synthèse totale de polypeptides est de plus en plus utile pour la préparation de protéines de structures bien définies ou portant des modifications naturelles, comme les modifications post-traductionnelles, ou non naturelles. Les méthodes de ligation chimique apportent une réponse à ce besoin, cependant elles s'avèrent limitées dans leur emploi et leur application industrielle.

De manière générale, dans ces méthodes, on souhaite que le lien entre les polypeptides assemblés par ligation soit natif, c'est-à-dire corresponde à la structure naturelle des polypeptides.

La principale méthode de ligation native existant à ce jour est celle de Kent et Dawson, décrite par exemple dans les demandes internationales WO 96/34878 et WO 98/28434. Cette méthode est fondée sur une réaction chimiosélective entre un peptide thioester (en C terminal) et un cystéinyl-peptide. Néanmoins, le principal inconvénient de cette méthode est la fabrication des peptides thioesters qui nécessite des procédés chimiques complexes. Ces méthodes ne peuvent pas empêcher la compétition entre les réactions des différents thioesters, conduisant inévitablement à des mélanges pouvant être difficilement séparables, donc affectant la pureté du produit final obtenu, et à des pertes inévitables de rendement.

Une méthode alternative est la ligation dite de Staudinger, décrite dans les demandes internationales WO 01/68565 et WO 01/87920. Celle-ci comprend la réaction d'un phosphinothioester avec un azoture et l'hydrolyse des réactifs combinés pour former une liaison amide. Cependant cette méthode est difficilement applicable à l'échelle industrielle.

Une autre méthode, décrite dans la demande internationale WO 2007/037812, repose sur la réaction d'un α-cétoacide avec une N-alkoxyamine dans une réaction de condensation décarboxylative. Toutefois, les cétoacides sont des molécules qui sont difficiles à fabriquer et à incorporer dans des peptides. Aussi, cette troisième méthode est difficilement applicable dans les laboratoires de synthèse peptidique ne disposant pas des moyens de réaliser des synthèses organiques complexes.

La publication de O. Melnyk et coll., Org. Lett., 12(22), 5238-41 (2010) et la demande FR-2952058, ainsi que la publication de Hou, W., Zhang, X., Li, F. & Liu, C.F. Peptidyl N,N-Bis(2-mercaptoethyl)-amides as Thioester Precursors for Native Chemical Ligation. Org. Lett. 13, 386-389 (2011) décrivent la ligation native de peptides au moyen de fragments peptide-bis(sulfanyléthyl)amino. Cependant cette méthode n'a jamais été utilisée, à ce jour, pour la synthèse de peptides par assemblage de 3 fragments ou plus
La demande WO2011/058188 décrit un artifice de purification consistant en l'introduction en fin de synthèse peptidique en phase solide d'un bras N-terminal muni d'une fonction azoture. Une réaction de Staudinger-Bertozzi ou une cycloaddition (CuAAC, SPAAC) permet de greffer le peptide cible sur une résine hydrocompatible préalablement fonctionnalisée par une phosphine ou un alcyne. Le peptide est finalement relargué par coupure du bras dans des conditions douces (base, nucléophile, ou photoirradiation) après lavages de la résine pour se débarrasser des peptides tronqués n'ayant pas pu se lier de façon covalente avec la résine. Toutefois, il n'est nullement envisagé dans ce document d'utiliser les moyens permettant le greffage pour réaliser la synthèse de protéines par ligation successive de peptides.

L'article M.Villain et al., Chemistry and Biology 8 (2001) 673-679 décrit un procédé de purification comportant une étape de greffage sur une résine, en fin de synthèse peptidique, du peptide comportant une cystéine ou une thréonine en extrémité N-terminale, par réaction du résidu N-terminal avec une fonction aldéhyde, de façon à former un cycle thiazolidine ou oxazolidine. Le peptide greffé est alors lavé puis décroché de la résine. Toutefois, il n'est nullement envisagé dans ce document d'utiliser les moyens permettant le greffage pour réaliser la synthèse de protéines par ligation successive de peptides.

Les documents US7884182 et Synthesis of Peptide-PNA-Peptide Conjugates by Semi-Solid-Phase Chemical Ligation Combined with Deactivation/Capture of Excess Reactants, Martijn C. de Koning. Eur. J. Org. Chem. 2004, 850-857, décrivent la ligation de peptides à l'aide d'un support solide. Les auteurs accrochent sur ce support un peptide H-Cys-A-SR (R = Alkyle) par formation d'un lien thiazolidine, puis effectuent une ligation native NCL entre la fonction thioester supportée et un peptide H-Cys-B. Les peptides H-Cys-A-SR sont difficiles à synthétiser. Cette méthode comporte un risque élevé d'avoir une réaction de polymérisation ou de cyclisation lors de la formation de la thiazolidine, puisque les deux extrémités sont réactives. Enfin, cette méthode ne permet pas de faire plus d'une ligation sur le support.

Le document FR 2 952 058 décrit un procédé de ligation de peptides en phase liquide, dans le sens C-terminal vers N-terminal. Ce procédé fait appel à :
- l'utilisation d'un peptide SEA,
- éventuellement l'utilisation d'un peptide SEAoff qui est ensuite converti en SEA.

Le document US 2002/0132975 décrit un procédé d'assemblage de peptides par ligation native séquentielle de fragments peptidiques en phase solide. La synthèse peut être faite de l'extrémité N-terminale vers C-terminale ou dans le sens inverse. Elle repose sur :
- l'utilisation de peptides portant une cystéine en N-terminal et un groupement COS- en C-terminal,
- la conversion en C-terminal du peptide supporté sur résine de la fonction - COS- en thioester-COSR,
- l'utilisation de la fonctionnalité thioester en C-terminal du peptide supporté sur résine pour réaliser chaque ligation sur la cystéine terminale du fragment peptidique suivant. Toutefois, les peptides thioacides (c'est-à-dire portant la fonction COS-), tels que ceux décrits comme produits de départ dans la demande US 2002/0132975, sont difficiles à préparer par synthèse, difficiles à purifier, et ils sont instables. En outre, l'étape d'activation de la fonction thioacide en thioester est délicate notamment lorsqu'il y a des cytéines libres dans la séquence peptidique, puisque le risque est d'alkyler aussi les cystéines. Enfin, le groupement thioacide, contrairement au groupement SEAoff, est un groupement réactif, non bloqué. Cela est illustré notamment dans la publication Canne, L. E. et al., J. Am. Chem. Soc. 1999, 121, 8720-8727, dans laquelle les auteurs ont constaté la formation significative d'un sous-produit cyclique provenant de la réactivité résiduelle du groupement thioacide.

Par rapport aux procédés de l'art antérieur, le procédé de l'invention bénéficie de la plus grande efficacité de conversion de la fonction SEAoff en thioester, les conditions de réaction sont plus douces et ne conduisent pas à la formation de sous-produits. En outre, SEAoff est un système bloqué dans les conditions de la ligation, contrairement à d'autres groupements réactifs, et notamment au thioacide décrit dans la demande US 2002/0132975. Enfin, les segments SEAoff sont synthétisés facilement en phase solide par la stratégie Fmoc/tert-butyle ce qui n'est pas le cas des peptides thioacides.

La transposition à l'échelle industrielle de procédés permettant de mettre en oeuvre des synthèses peptidiques par synthèse totale est un besoin qui requiert de trouver des procédés simples, peu coûteux, produisant des produits de qualité, de pureté élevée et raisonnables en matière d'hygiène industrielle.

Pour les raisons énoncées ci-dessus, il est devenu indispensable de trouver un procédé de synthèse totale, convergente et industrialisable, permettant de synthétiser un enchaînement peptidique de la longueur et de la nature souhaitée. Particulièrement un procédé mettant en jeu un assemblage du N-terminal vers le C-terminal, qui offre des qualités de simplicité de réalisation et de pureté des peptides ou polypeptides obtenus. En effet, l'assemblage du N-terminal vers le C-terminal offre un avantage considérable, comparé à la stratégie inverse et beaucoup plus classique d'assemblage du C-terminal vers le N-terminal, car dans ce cas le procédé permet une « auto-purification » des fragments peptidiques en se débarrassant des peptides N-tronqués acétylés, contaminants majeurs de la synthèse peptidique sur phase solide (SPPS).

La présente invention permet de surmonter les difficultés liées aux ligations multiples en solution. Elle permet de synthétiser des protéines de très grande taille. Elle peut être facilement automatisée et extrapolée à l'échelle industrielle.

### DESCRIPTION DE L'INVENTION

Il a été trouvé, ce qui fait l'objet de la présente invention, que l'assemblage de multiples fragments peptidiques dans une méthode de synthèse en phase solide mettant en jeu des méthodes simples comme la formation de peptides-thioesters et la ligation native, pouvait conduire à un procédé de synthèse totale, convergente, automatisable, industrialisable et répondant aux critères de pureté requis.

A cette fin, la présente invention propose un procédé d'assemblage de fragments peptidiques pour fabriquer un polypeptide comportant n fragments peptidiques et au moins n-1 acides aminés portant une fonction thiol, représenté par la formule :

A₁-C₁-A₂-C₂-A₃-...-Cᵢ₋₁Aᵢ-....-Cₙ₋₁-Aₙ (I)

dans laquelle A₁, A₂, A₃, ... Aᵢ ... , Aₙ sont des fragments peptidiques,
C₁, C₂, C₃ ... Cᵢ₋₁ ... Cₙ₋₁ sont des restes d'acides aminés portant une fonction thiol,
n est compris entre 3 et 50,
de préférence entre 3 et 20,
ou de manière encore préférée entre 3 et 10, et
i est un nombre entier quelconque compris entre 2 et n.

Ce procédé fait intervenir :
(a1) au moins une étape de préparation d'un fragment Y-A₁-SEAoff (II₁) dans lequel A₁ représente un fragment peptidique dont le C-terminal porte un groupement bis(2-sulfanyléthyl)amino cyclique 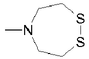 appelé SEAoff, et
   Y est un fragment capable de réagir avec une fonction d'un support solide de façon à former une liaison entre A₁ et un support solide,
(b) au moins une étape de réaction de Y-A₁-SEAoff (II₁) avec un support solide noté □-Y', □ représentant le support solide lui-même et Y' représentant une fonction réactive capable de réagir avec Y pour former un groupement Z suivant le schéma : 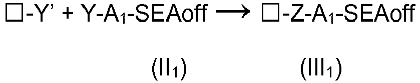
(a2) au moins une étape de préparation d'un fragment H-C₁(PG₁)-A₂-SEAoff (II₂) dans laquelle C₁, A₂ et SEAoff sont définis comme ci-dessus et (PG₁) représente H ou un groupement protecteur du thiol de l'amino-acide C₁,
(c1) au moins une étape de préparation d'un peptide-thioester de formule (III₁') à partir du bis(2-sulfanyléthyl)amino peptide □-Z-A₁-SEAoff (III₁) suivant le schéma : 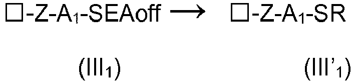 par action d'un thiol R-SH, éventuellement en présence d'un agent réducteur des disulfures cycliques, R pouvant être un radical alkyle ou aryle éventuellement substitué,
(d1) au moins une étape de condensation de (III'₁) sur le fragment peptidique (II₂) en présence d'un thiol aromatique ArSH dans des conditions où PG₁ est éliminé lorsqu'il est différent de H: 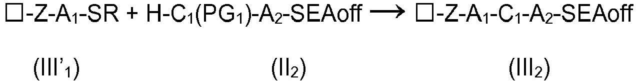
   (an-1) au moins une étape de préparation d'un fragment Cₙ₋₁(PGₙ₋₁)-An où (PGₙ₋₁) représente H ou un groupement protecteur du thiol de l'amino-acide Cₙ₋₁,
   (dn-1) Au moins une étape de condensation de Cₙ₋₁(PGₙ₋₁)-An avec

      □-Z-A₁-C₁-A₂-...Cᵢ₋₁Aᵢ-...Cₙ₋₂Aₙ₋₁SEAoff (IIIₙ₋₁)

      en présence d'un thiol aromatique ArSH dans des conditions où PGₙ₋₁ est éliminé lorsqu'il est différent de H pour donner :

      □-Z-A₁-C₁-A₂-...Cᵢ₋₁Aᵢ-...Cₙ₋₁Aₙ (IVₙ),

      ledit procédé comprend en outre, pour tout i = 1, ..., n-2,
(ci) au moins une étape de transformation de

   □-Z-A₁-C₁-...Cᵢ₋₁AᵢSEAoff (IIIᵢ)

   en

   □-Z-A₁-C₁-...Cᵢ₋₁-Aᵢ-SR (III'ᵢ)

   par action d'un thiol R-SH, éventuellement en présence d'un agent réducteur des disulfures cycliques,
   (di) au moins une étape de condensation de Cᵢ(PGᵢ)Aᵢ₊₁SEAoff où (PGᵢ) représente H ou un groupement protecteur du thiol de l'amino-acide Cᵢ,
   avec

   □-Z-A₁-C₁-...Cᵢ₋₁Aᵢ-SR (III'ᵢ)

   pour donner

   □-Z-A₁-C₁-...CᵢAᵢ₊₁SEAoff (IIIᵢ₊₁)

Selon un mode de réalisation particulier de l'invention, le procédé comporte encore :
(e) une étape de décrochage du peptide A₁-C₁-A₂-...Cᵢ₋₁Aᵢ-...Cₙ₋₁Aₙ (I) du support solide.

Selon un mode de réalisation de l'invention, le support solide □ est choisi parmi les résines, en particulier parmi les résines à base de polystyrène, polyacrylamide, polyéthylèneglycol, cellulose, polyéthylène, polyester, latex, polyamide, polydiméthylacrylamide, les polymères hydrophiles synthétiques ou naturels, les billes de verre, les gels de silice.

Selon un mode de réalisation de l'invention, C₁,...Cᵢ...Cₙ sont des cystéines.

Selon un mode de réalisation de l'invention, PG₁,... PGᵢ... PGₙ sont des groupements terbutyle sulfényle
Selon un mode de réalisation de l'invention,
Y' comporte une fonction choisie parmi un azoture, et Y est choisi parmi les groupements comportant une fonction alcyne, ou
Y' comporte une fonction alcyne et Y est choisi parmi les groupements comportant une fonction azoture, ou
Y' comporte une fonction aldéhyde, Y est H et l'acide aminé N-terminal de A₁ est choisi parmi une cystéine une sérine ou une thréonine, ou
Y' comporte une fonction aldéhyde, Y comporte un groupement NH₂ capable de former une base de Schiff
Selon un mode de réalisation de l'invention, R est choisi parmi un radical alkyle comprenant de 1 à 12 atomes de carbone, linéaire ou ramifié, éventuellement substitué, aralkyle ou aryle en C6-C12, éventuellement substitué.

Selon un mode de réalisation de l'invention, pour tout i = 2,...n-2 , les étapes di) du procédé sont mises en oeuvre dans des conditions ou PGᵢ est enlevé sélectivement *in situ,* sans affecter SEAoff et le solvant de la réaction est un tampon aqueux, de pH compris entre 4 et 9, contenant un thiol aromatique.

Un autre objet de l'invention est un support solide greffé par un peptide et répondant à la formule (III₁) ci-dessous :

□-Z-A₁-SEAoff (III₁)

dans laquelle A₁ représente un fragment peptidique, dont le C-terminal porte un groupement bis(2-sulfanyléthyl)amino cyclique 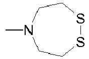 appelé SEAoff, □ représente un support solide et Z représente un groupement de liaison entre A₁ et □.

Un tel support solide peut être utilisé dans un kit de synthèse de polypeptides. Un tel kit de synthèse de polypeptides comprend habituellement au moins un support doté d'au moins une zone de réception sur ou dans laquelle est placé au moins un support solide greffé (III₁).

Un autre objet de l'invention est un dispositif automatisé pour la synthèse de peptides permettant de mettre en oeuvre de façon automatisée le procédé de synthèse de l'invention. Un tel dispositif comporte au moins un réservoir dans lequel est placé un support solide greffé par un peptide et répondant à la formule (III₁) ci-dessous :

□-Z-A₁-SEAoff (III₁)

Il comporte également des réservoirs distincts contenant :
- des peptides (IIi) Cᵢ(PGᵢ)Aᵢ₊₁SEAoff qui ont été décrits ci-dessus, ainsi que :
- au moins un thiol R-SH, et éventuellement un agent réducteur des disulfures cycliques
- les activateurs de couplage et
- les produits de lavage.

Un tel automate comporte également des moyens mécaniques de prélèvement et de distribution d'échantillons de produits, ainsi que des moyens informatiques permettant la mise en oeuvre contrôlée de ces moyens mécaniques.

La demande telle que déposée décrit un support solide greffé par un polypeptide comportant n fragments peptidiques et au moins n-1 acides aminés portant une fonction thiol et répondant à la formule (IVₙ) ci-dessous :

□-Z-A₁-C₁-A₂-...Cᵢ₋₁Aᵢ-...Cₙ₋₁Aₙ (IVₙ)

Un tel support solide peut être utilisé pour réaliser des tests d'affinité entre un polypeptide et une autre molécule.

Pour la mise en oeuvre de ces supports solides greffés dans des tests d'affinité biologique, il est généralement souhaité que la chaîne peptidique A₁-C₁-A₂-...Cᵢ₋₁Aᵢ-...Cₙ₋₁Aₙ soit repliée, de façon à présenter une conformation native biologiquement relevante. Pour cela, la chaîne est repliée après l'étape de synthèse, dans des conditions ou généralement les cystéines s'apparient pour former des ponts disulfure.

La demande telle que déposée décrit un kit de test biologique comportant au moins un support doté d'au moins une zone de réception sur ou dans laquelle est placé au moins un support solide greffé par au moins un polypeptide comportant n fragments peptidiques et n-1 acides aminés portant une fonction thiol et répondant à la formule (IVₙ) ci-dessous :

□-Z-A₁-C₁-A₂-...Cᵢ₋₁Aᵢ-...Cₙ₋₁Aₙ (IVₙ)

Un autre objet encore de l'invention est un procédé de fabrication d'un médicament comprenant au moins :
- la fabrication d'au moins un polypeptide selon le procédé défini ci-dessus, et
- son association avec un support pharmaceutiquement acceptable.

### DESCRIPTION DE MODES DE REALISATION DE L'INVENTION

L'invention est maintenant décrite plus en détails et de façon non limitative dans la description qui suit.

Par « peptides ou polypeptide » on entend, dans le cadre de la présente demande, une chaîne linéaire de résidus d'acides aminés (en nombre supérieur ou égal à deux) reliés par des liaisons peptidiques. Les « peptides ou polypeptides » au sens de la présente demande peuvent donc par exemple être des oligopeptides, peptides ou protéines selon l'acception conventionnelle de ces termes. Les résidus d'acides aminés présents dans les polypeptides selon l'invention peuvent être choisis parmi les résidus d'acides aminés protéinogéniques ou non. De préférence, ils sont choisis parmi les vingt résidus d'acides aminés protéinogéniques.

La notation des polypeptides s'effectue de l'extrémité N-terminale vers l'extrémité C-terminale. Les résidus d'acides aminés présents le long de la chaîne polypeptidique sont notés selon le code usuel à une lettre ou à trois lettres. Un résidu d'acide aminé est un fragment de polypeptide de formule -NH-(CH-R)-(C=O)-, dans laquelle R représente une chaîne latérale, différente d'un acide aminé à un autre.

Par « fragment peptidique » on entend, dans le cadre de la présente demande, une portion de polypeptide comprenant au moins un résidu d'acide aminé. Un fragment peptidique, au sens de la présente demande, peut donc être par exemple : une séquence de résidus d'acides aminés (telle que -AHG- ou -Ala-His-Gly-) si le fragment peptidique ne comprend ni l'extrémité N-terminale ni l'extrémité C-terminale du polypeptide ; ou bien une séquence de résidus d'acides aminés présentant un groupement à son extrémité N-terminale (telle que H-AHG- ou H-Ala-His-Gly-) si le fragment peptidique comprend l'extrémité N-terminale du polypeptide ; ou bien une séquence de résidus d'acides aminés présentant un groupement à son extrémité C-terminale (telle que -AHG-OH ou -Ala-His-Gly-OH) si le fragment peptidique comprend l'extrémité C-terminale du polypeptide.

Il est entendu que chacun des fragments peptidiques comprend de préférence uniquement des résidus d'acides aminés choisis parmi les 20 résidus d'acides aminés protéinogéniques. Toutefois, selon un mode de réalisation particulier, les fragments peptidiques Aᵢ en position interne de la séquence peuvent aussi comprendre un ou plusieurs résidus d'acides aminés non-protéinogéniques, l'un ou plusieurs des fragments peptidiques A₂ ... Aᵢ ... Aₙ₋₁ pouvant porter un ou plusieurs amino acides modifiés, la modification étant mise en place préalablement à la mise en oeuvre du procédé. A titre d'exemple, non limitatif, la modification des acides aminés peut être notamment choisie parmi des radicaux choisis parmi des résidus d'acides carboxyliques (biotine, groupement acétyle, résidu aminooxyacétique, un fluorophore comme la tetraméthylrhodamine, un agent de chélation des métaux comme l'acide 1,4,7,10-tétraazacyclododecane-1,4,7,10-tétraacétique (DOTA), un lipide comme l'acide palmitique, un polymère comme par exemple l'alpha-méthoxy-omega-carboxy poly(éthylène glycol) ou d'autres. Elle peut encore être choisie parmi des modifications post-traductionnelles d'acides aminés protéinogéniques connues de l'homme de l'art (méthylation, phosphorylation, acétylation, glycosylation, sulfatation, hydroxylation, carboxyméthylation, portant les protections appropriées pour l'incorporation en phase solide, etc...), ou éventuellement un médicament (la protéine servant alors de vecteur).

La présence d'acides aminés modifiés permet notamment de fonctionnaliser le polypeptide en vue de la détection d'interactions du polypeptide avec d'autres molécules (marqueurs de fluorescence...), mais d'autres types de fonctionnalisations peuvent être prévus.

Selon une manière simple, l'introduction d'une modification peut être effectuée en utilisant un acide aminé non-protéinogénique, notamment un dérivé d'acide aminé protéinogénique, pour introduire la modification lors de la synthèse en phase solide du fragment considéré. A titre d'exemple il est possible d'utiliser une Fmoc-L-Lys(Biotine)-OH, c'est à dire une lysine portant sur sa chaîne latérale une biotine, pour effectuer la synthèse d'un fragment. Ce fragment est ensuite engagé dans l'assemblage de manière identique à celle qui emploie les fragments d'acides aminés protéinogéniques.

Les résidus d'acides aminés des polypeptides A₁,...Aᵢ, Aₙ peuvent éventuellement être protégés de façon provisoire ou permanente par des groupements protecteurs des fonctions portées par les chaînes latérales, dont la nature varie en fonction des fonctions réactives des chaînes latérales et qui sont bien connues de l'homme du métier.

Selon un mode de réalisation de l'invention, un fragment peptidique Aᵢ comprend entre 2 et 600 résidus d'acides aminés, de préférence entre 5 et 100 résidus d'acides aminés, de manière plus particulièrement préférée entre 8 et 50 résidus d'acides aminés.

Le polypeptide de formule (II) peut être par exemple obtenu par une méthode de synthèse peptidique usuelle, notamment une méthode de synthèse en phase solide. Il peut également être obtenu au moyen d'une ligation native précédente.

La préparation du bis(2-sulfanyléthyl)amino peptide : Y-A₁-SEAoff (étape a1) du procédé) peut être effectuée selon la méthode décrite par Melnyk, O. et coll., Bis(2-sulfanyléthyl)amino native peptide ligation., Org. Lett., 12(22), 5238-41(2010), ainsi que dans FR-2952058. Selon la réalisation de ce procédé, l'acide aminé C-terminal est couplé à un support de résine polymère P greffé SEA (bis(2-sulfanyléthyl)amino) au moyen de la mise en présence d'un support de résine polymère greffé SEA avec un halogénure d'acide aminé ou avec un acide aminé et un agent d'activation, choisi de préférence parmi le PyBOP® (benzotriazol-1-yl-oxytripyrrolidinophosphonium hexafluorophosphate), le BOP, le PyBroP® (bromo-tris-pyrrolidino-phosphonium hexafluorophosphate), ou encore l'HATU (2-(1H-7-azabenzotriazol-1-yl)-1,1,3,3-tétramethyl uronium hexafluorophosphate methanaminium) et de manière plus particulièrement préférée le PyBroP et l'HATU. Cette réalisation est décrite plus en détails ci-après dans les exemples de préparation des fragments peptidiques de départ. A₁-SEA-P est ensuite fonctionnalisé par un groupement Y puis décroché du polymère P.

Le procédé de l'invention comporte la réaction d'un peptide fonctionnalisé Y-A₁-SEAoff (II₁) avec un support solide noté □-Y', □ représentant le support solide lui-même et Y' représentant une fonction réactive capable de réagir avec Y pour former un groupement Z suivant le schéma 1 :

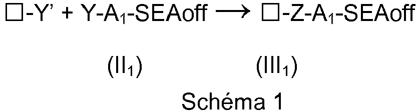

Le support solide est de préférence un polymère sous forme de particules (billes) insolubles ou solubles. On peut par exemple utiliser des résines à base de polystyrène, polyacrylamide, polyéthylèneglycol, cellulose, polyéthylène, polyester, latex, polyamide, polydiméthylacrylamide et des résines dérivées de celles-ci. Il est également possible d'utiliser un gel de silice ou des billes de verre à titre de support solide.

Avantageusement, on utilise un support solide choisi parmi les polymères hydrophiles synthétiques tels que les résines PEGA, ChemMatrix®, SPOCC (superpermeable organic combinatorial chemistry ou chimie combinatoire organique superperméable) ou des polymères carbohydrates naturels tels que de l'agarose ou du sépharose.

Ces résines sont greffées par un groupement Y' comportant une fonction choisie parmi un azoture, un alcyne, de préférence un alcyne vrai ou alcyne terminal, un cyclooctyne, Y est alors lui-même choisi parmi un alcyne, de préférence un alcyne vrai ou alcyne terminal, et un azoture.

Selon une variante ces résines sont greffées par un groupement Y' qui comporte une fonction aldéhyde, Y est H et l'acide aminé N-terminal de A₁ est choisi parmi une cystéine, une sérine ou une thréonine, ou un groupement NH₂ capable de former une base de Schiff, comme le groupement hydrazinocarbonyle H2NNHCO, ou plus généralement un dérivé de l'hydrazine ou H₂NO, c'est-à-dire un dérivé de l'hydroxylamine.

De façon plus précise, les modes de mise en oeuvre préférés de l'invention ont les suivants :
Quand Y' comporte un alcyne, Y comporte un azoture,
Quand Y' comporte un azoture, Y comporte un alcyne,
Quand Y' est un aldéhyde, Y est H et l'acide aminé N-terminal de A₁ est choisi parmi une cystéine, une sérine ou une thréonine
   ou
Quand Y' est un aldéhyde, Y est un groupement NH₂ capable de former une base de Schiff, comme le groupement hydrazinocarbonyle H2NNHCO, ou plus généralement un dérivé de l'hydrazine ou H₂NO, c'est-à-dire un dérivé de l'hydroxylamine.

La liaison entre le fragment peptidique et le support solide est effectuée par l'intermédiaire d'un groupement fonctionnel approprié, dit lieur (ou « linker »). Ainsi, dans un premier temps le fragment peptidique N-terminal est fixé sur les groupements fonctionnels lieurs du support solide (en laissant l'extrémité C-terminale du fragment peptidique sous forme de SEAoff) par l'intermédiaire de l'acide aminé correspondant à l'extrémité N-terminale du polypeptide à synthétiser, ce qui constitue la première amorce, puis les fragments peptidiques suivants sont ajoutés selon la succession de réactions décrite ci-dessous.

Selon une première variante, la préparation du support solide greffé par le bis(2-sulfanyléthyl)amino peptide : □-Z-A₁-SEAoff (III₁) peut être effectuée selon la méthode décrite dans WO2011/058188 :

On fait réagir le peptide A₁-SEAoff avec un composé répondant à la formule générale X₁-L-X₂, dans laquelle X₁ représente un groupement choisi parmi :

-N₃

et

-C≡CH

L représente un groupement espaceur et X₂ représente un groupement comportant un groupe fonctionnel capable de réagir avec le groupement NH₂ terminal du peptide A₁, la liaison entre X₂ et A₁ étant résistante dans les conditions de ligation mentionnées ci-dessus et susceptible d'être clivée dans des conditions qui ne dégradent pas la chaîne peptidique. De tels groupements X₂ sont décrits de façon détaillée dans WO2011/058188.

Le peptide A1 est supporté par une phase solide par l'intermédiaire de sa fonction acide terminale et d'un groupement précurseur de SEAoff. La mise en contact du peptide A₁ supporté avec le composé X₁-L-X₂ résulte en la formation d'une liaison covalente entre le groupement NH₂ terminal du peptide A₁ et le groupement X₂ pour former un composé X₁-L'-A₁ dans lequel X₁ a la même signification que ci-dessus et L' représente un bras de liaison produit de la condensation de -L-X₂ avec l'extrémité N-terminale du peptide A₁. Le groupement SEAoff n'est formé qu'après le détachement du peptide X₁-L'-A₁ du support solide.

De façon habituelle, L' est une chaine alcane di-yle comportant de 1 à 50 atomes de carbone, et une ou plusieurs fonctions qui peuvent être choisies, de façon non exhaustive, parmi : une fonction hydroxyle, une fonction amine, une fonction ester, thioester, une fonction éther, une fonction amide, une fonction NO₂, SO₂, un atome d'halogène, un groupement aryle éventuellement substitué, et cette chaîne alcane di-yle est éventuellement interrompue par un ou plusieurs groupements choisis parmi : un pont éther (-O-), un pont amine (-NH-), un pont ester (-COO-), un pont amide (-CONH-), un pont urée (-NH-CO-NH-), un pont uréthane (-NH-CO-O-).

Ensuite le groupement réactif X₁ réagit avec un support solide porteur d'une fonctionnalité appropriée. De tels supports sont décrits dans WO2011/058188. Notamment, lorsque X₁ représente :

-C≡CH

on peut choisir un support solide porteur de l'une des fonctionnalités suivantes

□-N₃

□-NH-CO-(CH₂)ₘ-N₃

Lorsque X₁ représente N₃, on peut par exemple choisir un support solide fonctionnalisé par un groupement alcyne, de préférence un alcyne vrai, présent dans une molécule plus ou moins complexe telle que celles illustrées dans WO2011/058188, comme par exemple :

□-NH-CO-(CH2)m-C≡C-H,

un groupement cyclooctyne... La réaction entre X₁ et le support solide est mise en oeuvre dans des conditions chimiosélectives et il ne se produit pas de réaction concurrente entre X₁ et les acides aminés du peptide A₁.

Selon une seconde variante, la préparation du support solide greffé par le bis(2-sulfanyléthyl)amino peptide : □-Z-A₁-SEAoff (III₁) peut être effectuée selon la méthode décrite dans M.Villain et al., Chemistry and Biology 8 (2001) 673-679.

Selon cette méthode, un lien thiazolidine ou oxazolidine est formé entre une fonction aldéhyde portée par le support solide et une cystéine, respectivement une sérine ou une thréonine, N-terminale du peptide A₁ suivant le schéma 2 ci-dessous :

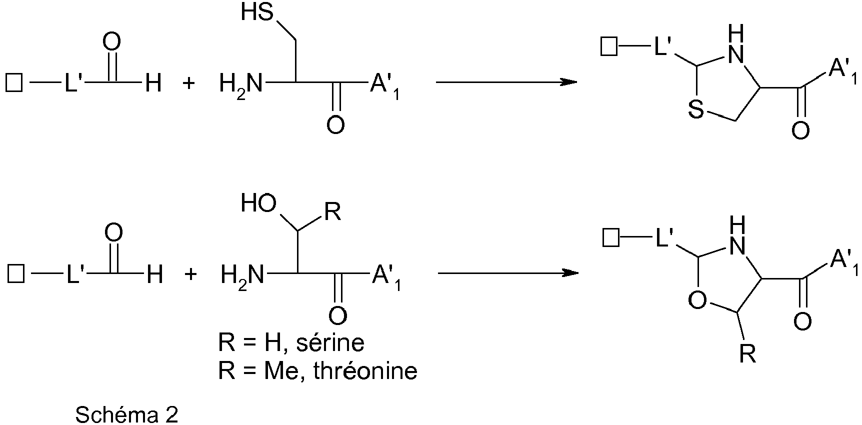

Dans ce schéma, L' représente un bras espaceur entre le support solide et la fonction aldéhyde, A'₁ représente le résidu peptidique qui forme avec la cystéine la sérine ou la thréonine N-terminale le peptide A₁. Avantageusement L' est une chaine alcane di-yle comportant de 1 à 50 atomes de carbone, et une ou plusieurs fonctions qui peuvent être choisies, de façon non exhaustive, parmi : une fonction hydroxyle, une fonction amine, une fonction ester, thioester, une fonction éther, une fonction amide, une fonction NO₂, SO₂, un atome d'halogène, un groupement aryle éventuellement substitué, et cette chaîne alcane di-yle est éventuellement interrompue par un ou plusieurs groupements choisis parmi : un pont éther (-O-), un pont amine (-NH-), un pont ester (-COO-), un pont amide (-CONH-), un pont urée (-NH-CO-NH-), un pont uréthane (-NH-CO-O-).

En effet, selon cette variante, le greffage peut être fait par une réaction directe entre un peptide et le support solide fonctionnalisé. La seule obligation consiste en la présence en
position N-terminale du peptide A₁ d'un résidu cystéine, sérine ou thréonine. Soit le peptide final visé comporte l'un de ces acides aminés en position N-terminale, et la méthode peut être mise en oeuvre sur ce peptide sans modification de séquence. Sinon, il faut prévoir l'ajout de l'un de ces trois résidus en position N-terminale de la séquence cible.

Dans tous les cas, le bras de liaison Z reliant le support solide et A1 doit être résistant dans les conditions de réalisation des étapes de transformation ci) et de condensation di). Par ailleurs il doit pouvoir être clivé dans des conditions permettant de libérer le polypeptide (I) sans l'endommager.

L'invention repose encore sur la préparation de fragments H-Cᵢ₋₁(PGᵢ₋₁)-Aᵢ-SEAoff (IIᵢ) représentés ci-dessous (avec i=2,...n-1) dans lesquels Cᵢ₋₁, Aᵢ et SEAoff sont définis comme ci-dessus et (PGᵢ₋₁) représente H ou un groupement protecteur du thiol de l'amino-acide Cᵢ₋₁.

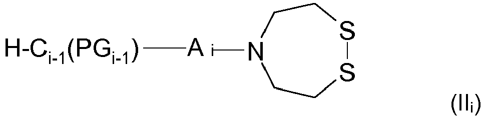

Le peptide de formule (IIᵢ) comprend un atome d'hydrogène et un résidu Cᵢ₋₁(PGᵢ₋₁) à l'extrémité N-terminale. Le résidu Cᵢ₋₁ est un résidu d'acide aminé comportant une fonction thiol. Cette fonction thiol peut être en particulier une fonction béta-amino thiol (auquel cas le résidu Cᵢ₋₁ représente de préférence le résidu cystéine), une fonction gamma-amino thiol (auquel cas le résidu Cᵢ₋₁ représente de préférence le résidu homocystéine), ou une selenocystéine.

Dans toute la description qui suit, selon un mode de réalisation particulier, Cᵢ₋₁ peut se lire comme représentant un résidu cystéine (Cys).

Suivant une variante préférée de l'invention, la fonction thiol de Cᵢ₋₁ est protégée par un groupe protecteur PGᵢ₋₁. Avantageusement PGᵢ₋₁ représente un groupement protecteur (SR') de façon à former un reste disulfure sur le thiol de l'amino-acide Cᵢ₋₁, et R' représente un groupement alkyle en C₁-C₆, en particulier SR' représente un groupement terbutyle sulfényle, ou un groupement aryle sulfényle ou aralkyle sulfényle en C₆-C₁₂, tel que phényle sulfényle ou benzyle sulfényle.

Il est entendu que chaque fragment peptidique Aᵢ peut avoir été préparé préalablement par une suite d'opérations telles que décrites ci-dessus, selon le procédé de l'invention ou par toute autre méthode de synthèse de peptides.

L'invention repose sur la transformation ci) de peptides greffés sur un support solide et porteurs d'une fonction SEAoff en peptide-thioesters, □-Z-A1-C1-A2-...Ci-2-Ai-1-SR (i=2 ,...n) (III'i-1) et leur réaction de condensation di) sur un peptide (ou polypeptide) portant une fonction bis(2-sulfanyléthyl)amino à l'état de disulfure cyclique, répondant à la formule générale : 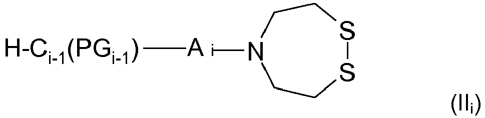 dans laquelle Aᵢ représente un fragment peptidique dont le C-terminal porte un groupement bis(2-sulfanyléthyl)amino cyclique 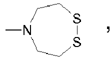 appelé SEAoff, et
Cᵢ₋₁ représente un résidu d'acide aminé portant une fonction thiol.
Il est entendu que le terme SEAoff désigne le disulfure cyclique non réactif, par opposition au disulfure réactif de structure -N[(CH₂)₂-SH]₂ ci-après appelé SEAon.
Cette étape particulière a été décrite pour son application à d'autres composés dans Dheur, J., Ollivier, N., Vallin, A. & Melnyk, O. Synthesis of Peptide Alkylthioesters Using the Intramolecular N,S-Acyl Shift Properties of Bis(2-sulfanylethyl)amido Peptides. J. Org. Chem. 76, 3194-3202 (2011)
Cette étape de synthèse repose sur au moins une étape ci) (avec i=2,...n-1) de transformation de

□-Z-A₁-...Cᵢ₋₁Aᵢ-SEAoff (IIIᵢ)

en

□-Z-A₁-...Cᵢ₋₁-Aᵢ-SR (III'ᵢ)

par action d'un thiol R-SH, éventuellement en présence d'un agent réducteur de disulfures cycliques.

Il est entendu que lorsque i=n, la transformation de SEAoff en SR n'est pas nécessaire et la réaction de condensation dn) de (IIIₙ₋₁) avec le fragment peptidique C-terminal -Cₙ₋₁(PGₙ₋₁)Aₙ peut être mise en oeuvre directement.

Cette transformation est suivie d'une étape de condensation di) de

□-Z-A₁-...Cᵢ₋₁Aᵢ-SR (III'ᵢ)

avec Cᵢ(PGᵢ)-Aᵢ₊₁-SEAoff (IIᵢ₊₁) en présence d'un thiol aromatique ArSH dans des conditions où PGᵢ, lorsqu'il est différent de H, est éliminé pour donner :

□-Z-A₁-...Cᵢ₋₁Aᵢ-CᵢAᵢ₊₁-SEAoff (III'ᵢ₊₁)

Dans le cas où i=n-1, l'étape de condensation est faite avec un peptide -Cₙ₋₁(PGₙ₋₁)Aₙ non porteur du groupement SEA, la transformation de SEAoff en SR n'est pas nécessaire mais peut être opérée, notamment dans le cas où l'ensemble de la synthèse est mis en oeuvre à l'aide d'un automate, le reste des conditions opératoires étant les mêmes que pour les autres condensations, notamment en présence d'un thiol aromatique ArSH dans des conditions où PGₙ₋₁, lorsqu'il est différent de H, est éliminé.

Le radical alkyle représenté par R est un radical alkyle de 1 à 12 atomes de carbone, linéaire ou ramifié, ou aryle en C6-C12 éventuellement substitué par un ou plusieurs groupements choisis parmi les atomes d'halogène (F par exemple), ou les radicaux CO₂H, SO₃H, CONH₂, OH, SH, alkyloxy, alkylthio, mercaptoalkylthio, le reste d'un ester ou un reste polyéthylène glycol, ou par un phényle éventuellement substitué, ou d'autres groupements organiques qui n'interfèrent pas avec les réactions mises en oeuvre. Le thiol de formule générale R-SH employé dans l'étape ci) peut être par exemple HSCH₂CH₂CO₂H, HSCH₂CH₂SO₃H, HSCH₂CH₂SH, HSCH₂CH₂SCH₂CH₂SH ou HSCH₂Ph.

Selon une variante, si le thiol R-SH est suffisamment réducteur la présence d'un agent réducteur des disulfures cycliques supplémentaire n'est pas nécessaire.

Le thiol aromatique employé à l'étape di) est avantageusement choisi parmi des composés réducteurs des liaisons disulfure, de préférence choisi parmi le thiophénol et ou les dérivés obtenus par substitution du noyau aromatique, par exemple le 4-carboxyméthylthiophénol. De façon alternative, des thiols non aromatiques tels que le dithiothréitol, le benzylmercaptan,peuvent être utilisés à la place du thiol aromatique. Bien entendu, des mélanges de ces thiols peuvent aussi convenir.

Le réducteur de SEAoff employé à l'étape ci) peut être choisi parmi les réducteurs de disulfures cycliques. Il peut être notamment choisi parmi les phosphines (par exemple la tris (2-carboxyéthyl)phosphine), ou tout autre réducteur des disulfures cycliques comme les thiols (par exemple le dithiothreitol (DTT)).

De façon avantageuse, l'étape de condensation est faite en présence d'un excès de Cᵢ(PGᵢ)-Aᵢ₊₁-SEAoff ou de Cₙ₋₁(PGₙ₋₁)-Aₙ.

La réaction de ligation intervient de préférence en milieu aqueux, par exemple dans un tampon phosphate à un pH compris entre 4 et 9. De préférence, cette réaction est effectuée à un pH compris entre 5 et 9, avantageusement entre 5,5 et 8,5, de manière plus particulièrement préférée à un pH compris entre 5,5 et 7,5 et idéalement à un pH voisin de 7,0.

La réaction de condensation est effectuée de préférence à une température comprise entre 0 et 50°C, et idéalement à une température d'environ 37°C. La durée de la réaction est ajustée en fonction du choix des réactifs et des autres conditions de la réaction. La durée appropriée peut également être ajustée selon les résultats d'une analyse de chromatographie liquide - spectrométrie de masse au cours de la réaction. La durée appropriée sera typiquement de quelques heures à quelques jours.

Avantageusement, on procède à un lavage du support solide entre deux étapes de condensation, de façon à éliminer les résidus peptidiques en excès n'ayant pas réagi et qui peuvent être récupérés ou recyclés éventuellement.

Eventuellement, on procède à une déprotection des chaînes latérales des acides aminés composant le polypeptide à l'aide de moyens bien connus de l'homme du métier.

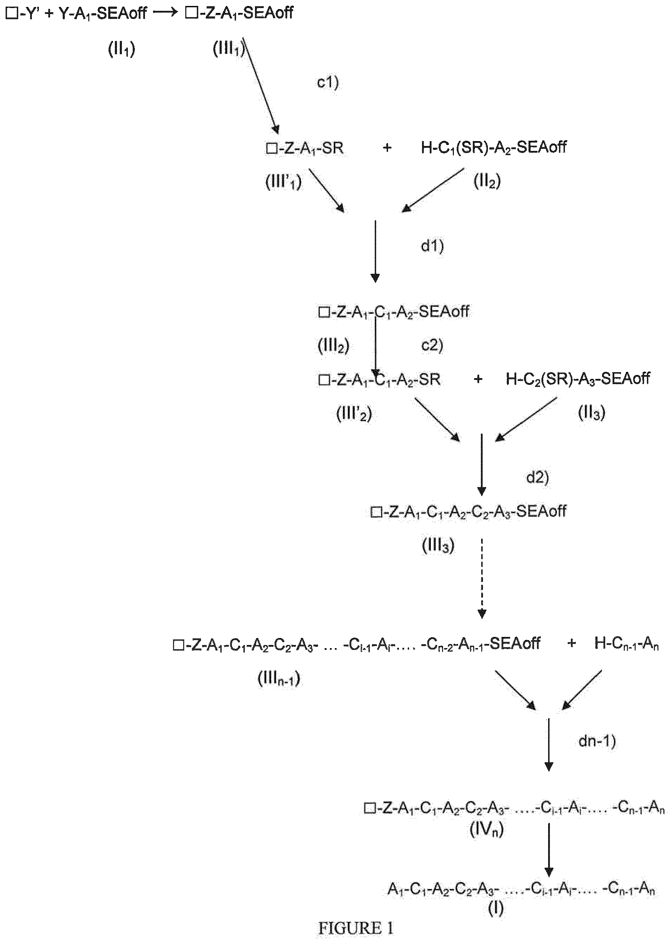

Selon l'invention, on peut représenter le procédé d'assemblage de multiples fragments peptidiques selon le schéma représenté sur la figure 1.

Selon l'invention, le procédé conduit à la préparation d'un assemblage peptidique multiple greffé sur un support solide □ par l'intermédiaire d'un groupe de liaison Z, l'assemblage peptidique comportant des restes d'acides aminés portant une fonction thiol, c'est à dire un assemblage de n fragments peptidiques Aᵢ et de au moins n-1 acides aminés Cᵢ portant une fonction thiol, représenté par la formule :

□-Z-A₁-C₁-A₂-C₂-A₃-...-Cᵢ₋₁-Aᵢ-....-Cₙ₋₁-Aₙ (IVₙ)

dans laquelle A₁, A₂, A₃, ... Aᵢ ... , Aₙ sont des fragments peptidiques,
C₁, C₂, C₃ ... Cᵢ₋₁ ... Cₙ₋₁ sont des restes d'acides aminés portant une fonction thiol,
n est compris entre 3 et 50,
de préférence entre 3 et 20,
ou de manière encore préférée entre 3 et 10, et
i est un nombre entier quelconque compris entre 2 et n.

Le procédé de l'invention comporte donc n-1 étapes d'assemblage peptidique. Avantageusement les n-1 étapes d'assemblage peptidique reposent sur une étape de transformation de □-Z-A₁-C₁-A₂-...Cᵢ₋₁Aᵢ-SEAoff en □-Z-A₁-C₁-A₂-...Cᵢ₋₁-Aᵢ-SR
par action d'un thiol R-SH, éventuellement en présence d'un agent réducteur de disulfures cycliques, suivie d'une étape de condensation de □-Z-A₁-C₁-A₂-...Cᵢ₋₁Aᵢ-SR avec Cᵢ(PGᵢ)-Aᵢ₊₁-SEAoff en présence d'un thiol aromatique ArSH dans des conditions où PGᵢ est éliminé pour donner : □-Z-A₁-C₁-A₂-...Cᵢ₋₁Aᵢ-CᵢAᵢ₊₁-SEAoff

Toutefois, il n'est pas exclu qu'une ou plusieurs étapes d'assemblage peptidique mise en oeuvres dans la synthèse du peptide (IVₙ) soient réalisées à l'aide d'une autre méthode de ligation connue de l'homme du métier.

En particulier, comme expliqué ci-dessus, l'étape n-1 peut être une ligation SEA, par réaction directe de SEAoff avec Cₙ₋₁(PGₙ₋₁)-Aₙ.

De façon avantageuse, toutes les étapes du procédé de production du peptide (IVₙ) sont mises en oeuvre suivant l'enchaînement d'étapes ci), di), i= 1, ...n-2, éventuellement cn-1), puis dn-1).

On peut aussi prévoir dans la fabrication du polypeptide et de préférence avant le décrochage de la résine une ou plusieurs étapes de traitement chimique, comme une transformation d'acides aminés, suivant des méthodes connues de l'homme du métier telles que par exemple l'alkylation d'une homocystéine en méthionine ou la désulfurisation d'une cystéine ou la désélénisation d'une sélénocystéine en alanine.

De façon préférentielle, le procédé est suivi d'une étape de coupure de la liaison entre le support solide et le peptide (I) dans des conditions permettant de libérer le peptide (I) :

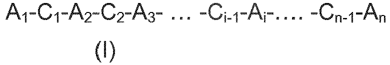

Suivant la nature du bras de liaison Z, les moyens de coupure seront différents. Notamment, suivant l'enseignement de WO2011/058188, on peut, en fonction de la nature du bras de liaison Z, libérer le peptide (I) par clivage de la liaison covalente entre la partie X₂ du bras espaceur Z et le peptide (I) par attaque nucléophile, ou dans des conditions alkalines, ou acides, ou par irradiation UV.

Lorsque le bras espaceur Z résulte de la condensation d'une cystéine d'une sérine ou d'une thréonine N-terminale du peptide A₁ avec une fonction aldéhyde portée par le support solide pour former un cycle thiazolidine, respectivement oxazolidine, le peptide final est avantageusement libéré par un traitement à l'aide de O-méthyl hydroxylamine, suivant l'enseignement de M.Villain et al., Chemistry and Biology 8 (2001) 673-679.

Ces dernière conditions sont également applicables au cas ou le lien Z formé est une base de Schiff.

Un autre objet de l'invention est un support solide greffé par un peptide et répondant à la formule ci-dessous :

□-Z-A₁-SEAoff (III₁)

Dans laquelle Z, □, A₁ et SEAoff ont la même signification que ci-dessus. De tels supports greffés par un premier fragment peptidique A₁ permettent d'amorcer la synthèse de polypeptides complexes. On peut notamment prévoir de préparer de façon industrielle ou semi-industrielle des supports solides greffés par des séquences de peptides A₁ d'intérêt général qui sont fréquemment employées comme séquence N-terminale de peptides d'intérêt biologique. Par exemple on peut citer les types de séquences suivants : peptides signaux pour l'adressage et la sécrétion de protéines, peptides marqueurs (de fluorescence par exemple), tags de purification tels que tags histidine, peptides porteur d'un signal d'ubiquitination etc...

Un tel support solide greffé par un premier peptide, en particulier un peptide d'intérêt peut avantageusement être utilisé dans un kit de synthèse de polypeptide. Dans un tel kit, il peut être associé avec un support comportant des zones de réception, telles que les puits d'une plaque multi puits dans lesquels des réactions de ligation successives peuvent être mises en oeuvre. Il peut être associé avec des réactifs de synthèse tels que ceux qui ont été décrits ci-dessus pour la réalisation des réactions de ligation.

La demande telle que déposée décrit un dispositif automatisé pour la synthèse de peptides permettant de mettre en oeuvre de façon automatisée le procédé de synthèse de l'invention. Un tel dispositif comporte au moins un réservoir dans lequel est placé un support solide greffé par un peptide et répondant à la formule (III₁) ci-dessous :

□-Z-A₁-SEAoff (III₁)

A1 peut notamment être choisi parmi les peptides d'intérêt qui ont été mentionnés ci-dessus.

Il comporte également des réservoirs contenant :
- des peptides (IIi) Cᵢ(PGᵢ)Aᵢ₊₁SEAoff qui ont été décrits ci-dessus, différents réservoirs étant prévus de façon à loger chaque peptide de séquence distincte.

On prévoit aussi des réservoirs pour :
- au moins un thiol R-SH, et éventuellement un agent réducteur des disulfures cycliques,
- les activateurs de couplage et
- les produits de lavage.

Un tel automate comporte également des moyens mécaniques de prélèvement d'échantillons dans les différents réservoirs et des moyens de distribution de ces échantillons dans le réservoir comportant le support solide. De tels moyens peuvent consister en un ensemble de tuyaux de circulation de fluides et valves, arrangés éventuellement en circuits microfluidiques, ou en des bras articulés équipés de pipettes de prélèvement. Le dispositif automatisé comporte aussi des moyens informatiques (logiciels) permettant la mise en oeuvre contrôlée de ces moyens mécaniques, permettant la mise en oeuvre de réactions séquencées.

La demande telle que déposée décrit un support solide greffé par un polypeptide comportant n fragments peptidiques Aᵢ et au moins n-1 acides aminés Cᵢ portant une fonction thiol et répondant à la formule (IVₙ) ci-dessous :

□-Z-A₁-C₁-A₂-...Cᵢ₋₁Aᵢ-...Cₙ₋₁Aₙ (IVₙ)

Dans laquelle Z, □, Aᵢ, Cᵢ, i, n et SEAoff ont la même signification que ci-dessus. En effet, à l'issue des ligations successives et de l'éventuelle déprotection des chaines latérales des acides aminés, et du repliement de la chaîne peptidique, le support solide peut être utilisé pour réaliser des tests biologiques, notamment des tests d'affinité des polypeptides synthétisés pour des molécules de toute nature.

La demande telle que déposée décrit un kit de test et/ou de diagnostic comportant au moins un support comportant au moins une zone de réception, sur laquelle est placé au moins un support solide greffé par au moins un polypeptide comportant n fragments peptidiques et au moins n-1 acides aminés porteurs d'une fonction thiol, le support solide greffé répondant à la formule (IVₙ) ci-dessous :

□-Z-A₁-C₁-A₂-...Cᵢ₋₁Aᵢ-...Cₙ₋₁Aₙ (IVₙ)

Ainsi, le kit de test peut être utilisé pour le screening de molécules biologiquement actives ou pour des tests de diagnostic. On peut prévoir que le kit de diagnostic comporte plusieurs zones de réception distinctes, sur lesquelles un support solide greffé par des polypeptides identiques ou distincts est déposé. Par exemple, des supports solides greffés par des polypeptides identiques ou distincts peuvent être placés dans les puits d'une plaque à puits de façon à former un support de test et/ou de diagnostic.

De préférence, pour la réalisation de tests, les polypeptides greffés sur le support solide sont préalablement mis en contact avec un milieu qui favorise la formation d'une structure bi- et/ou tridimensionnelle. Par exemple suivant l'enseignement de E.C.B.Johnson et al., Angew. Chem. Int. Ed. 2006, 45, 3283-3287, on peut mettre le polypeptide en contact avec un tampon phosphate de pH 8 et le soumettre à un dégagement d'air, ou on peut mettre le peptide en contact avec une solution aqueuse de pH égal à 9. Un autre mode utile pour le repliement est l'utilisation du couple GSH, GSSG (GSH pour glutation, GSSG pour glutation oxydé).

Le kit de tests et/ou de diagnostic peut encore comprendre des moyens de détection d'une interaction entre les polypeptides et les molécules testées.

Un autre objet encore de l'invention est un procédé de fabrication d'un médicament comprenant au moins :
- la fabrication d'au moins un polypeptide selon le procédé défini ci-dessus, et
- son association avec un support pharmaceutiquement acceptable.

En effet, le procédé de l'invention peut être mis en oeuvre dans le but de synthétiser des polypeptides ayant une activité thérapeutique, notamment des vaccins. Leur association avec un support pharmaceutiquement acceptable est réalisée par l'homme du métier à l'aide de ses connaissances générales et en fonction des propriétés du polypeptide (solubilité notamment) et du mode d'administration choisi.

D'autres caractéristiques et avantages de l'invention apparaîtront à la lecture de la description donnée à titre d'exemple et en référence au dessin annexé.

### FIGURES

Figure 1 : représentation schématique générale du procédé de synthèse de peptides suivant l'invention.
Figure 2: représentation schématique du procédé de synthèse du peptide **11a**, H-GILKEPVQGA-CHHLEPGG-CHHLEPAG-CILKEPVHGA-NH₂ suivant l'exemple I.
Figure 3 : Chromatogramme RP-HPLC du peptide **4**
Figure 4 : Spectre MS du peptide **4**
Figure 5 : Chromatogramme RP-HPLC du peptide **5.1a**
Figure 6 : Spectre MS du peptide **5.1a**
Figure 7 : Chromatogramme RP-HPLC du peptide **8.2**
Figure 8 : Spectre MS du peptide **8.2**
Figure 9 : Chromatogramme RP-HPLC du peptide **8.3**
Figure 10 : Spectre MS du peptide **8.3**
Figure 11 : Chromatogramme RP-HPLC du peptide **11a**
Figure 12 : Spectre MS du peptide **11a**
Figure 13 : Chromatogramme RP-HPLC du peptide **11a** purifié
Figure 14 : Chromatogramme RP-HPLC du peptide **16a**
Figure 15 : Spectre MS du peptide **16a**
Figure 16 : Chromatogramme RP-HPLC du peptide **19**
Figure 17 : Spectre MS du peptide **19**
Figure 18 : Chromatogramme RP-HPLC du peptide **21**
Figure 19 : Spectre MS du peptide **21**
Figure 20 : Chromatogramme RP-HPLC du peptide **23.1**
Figure 21 : Spectre MS du peptide **23.1**
Figure 22 : Chromatogramme RP-HPLC du peptide **26.1**
Figure 23 : Spectre MS du peptide **26.1**
Figure 24 : Chromatogramme RP-HPLC du peptide **29**
Figure 25 : Spectre MS du peptide **29**

### PARTIE EXPERIMENTALE

### I- SYNTHESE DU PEPTIDE 11 :

### H-GILKEPVQGA-CHHLEPGG-CHHLEPAG-CILKEPVHGA-NH₂

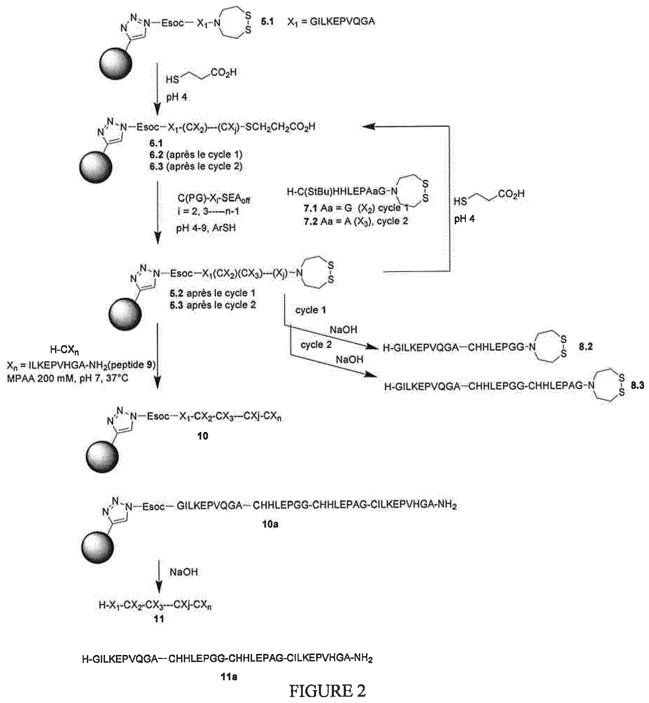

Le peptide **11a** est préparé par ligations successives de quatre fragments peptidiques suivant le schéma représenté sur la figure 2.

### I-A- Préparation de N₃-Esoc-GILKEPVQGA-SEAoff (peptide 4) :

Le groupement N₃-Esoc est décrit dans le document WO2011/058188: 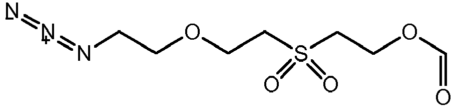 Le peptide nommé X₁ sur la figure 2 est GILKEPVQGA

La préparation de N₃-Esoc-GILKEPVQGA-SEAoff est illustrée sur le schéma 3 ci-dessous.

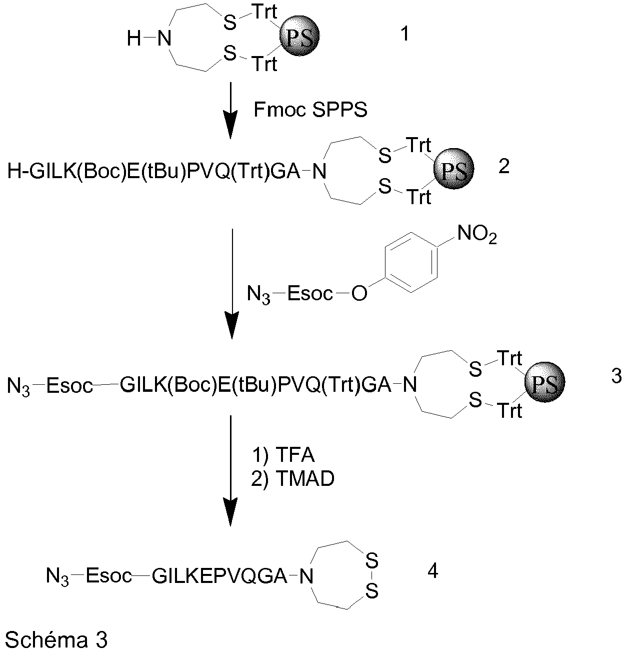

### Mode opératoire :

### - Synthèse de la peptidyl résine 2 H-GILK(Boc)E(tBu)PVQ(Trt)GA-SEA-PS

Le support solide SEA-PS (avec PS = polystyrène) est décrite dans la demande FR-2952058 Cette peptidyl résine est synthétisée à une échelle de 0.25 mmole en suivant le protocole décrit dans Ollivier, N.; Dheur, J.; Mhidia, R.; Blanpain, A.; Melnyk, O. Organic Letters 2010, 12, 5238-41.

Après synthèse, la résine est lavée successivement avec du CH₂Cl₂ (2 x 2 min) puis du DMF (2 x 2 min) et est engagée dans l'étape suivante.

### - Synthèse du fragment N₃-ESOC-GILKEPVQGA-SEAoff 4 :

Le bras activé N3-Esoc-ONp **(Schéma 3)** est synthétisé selon la procédure décrite dans WO2011/058188. Ce carbonate mixte de 2-[2-(2-azido-éthoxy)-éthylsulfonyle]-éthyle et de 4-nitrophényle (145 mg, 0.4 mmol, 1.5 eq) est dissous dans un minimum de DMF puis transféré directement sur la résine. De la *N*-méthylmorpholine (55 µL, 0.5 mmol, 2 eq) est additionnée. Le milieu réactionnel est agité 12 heures sous atmosphère d'argon à température ambiante. La résine est lavée successivement au DMF (2 x 2 min), CH₂Cl₂ (2 x 2 min) et Et₂O (2 x 2 min) puis séchée sous vide.

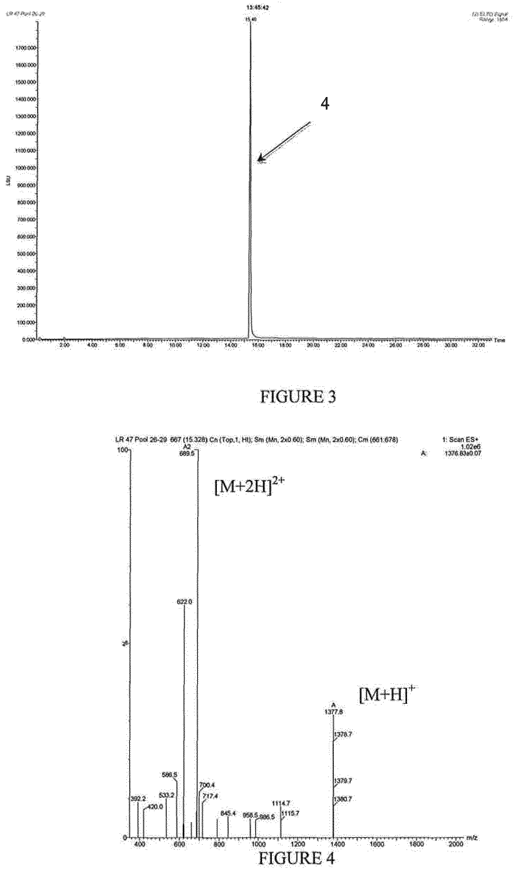

La déprotection finale des chaînes latérales et le décrochage du peptide de la résine sont réalisés par l'action d'un mélange TFA/TIS/DMSO/H₂O (20 mL, 92.5/2.5/2.5/2.5% en v/v) pendant 1h30. Le peptide est précipité dans un mélange froid d'éther diéthylique/heptane (200 mL, 1:1 v/v), centrifugé, puis dissous dans un minimum d'eau et lyophilisé.
Le peptide **4** brut (30 mg, 0.02 mmol) est dissous dans du tampon phosphate de sodium (20 mL, 0.2M, pH=7.2) puis oxydé en présence de *N*,*N*,*N'*,*N'*-tétraméthylazodicarboxamide (TMAD) (6.8 mg, 0.04 mmol, 2 eq) pendant 20 minutes. Le milieu réactionnel est purifié directement par chromatographie liquide haute performance en phase inverse (RP-HPLC) (Colonne C18 Nucléosil (d=1 cm, L=20 cm, 120 Å, 5 µm), détection UV (λ 215 nm), tampon A : H₂O/TFA (1:0.05% en v/v), tampon B : CH₃CN/H₂O/TFA (4:1:0.05% en v/v/v), gradient : tampon B (0 à 20% en 5 min puis 20 à 42% en 60 min, 6 mL/min)). 15 mg de peptide 4 sont obtenus (R= 14%). Le résultat de l'analyse est représenté sur la figure 3.
Analyse LC-MS :
LC-MS : Tampon A : H₂O/TFA (1:0.05% v/v), Tampon B : CH₃CN/H₂O/TFA (4:1:0.05% v/v/v). RP-HPLC sur une colonne C18 Xbridge BEH (300 Å, 3.5 µm, 4.6 x 150 mm) en utilisant un gradient linéaire 0-100% B en 30 min, débit 1 mL/min, détection ELS.
MS : Mode d'ionisation electrospray positif, voltage cône 30V, analyseur quadripolaire. Résultat illustré sur la figure 4.

### I-B- Préparation de la peptidyl résine P-1-(1,2,3-triazolyl)-Esoc - GILKEPVQGA -SEAoff, avec P = PEGA 800, 5.1

La préparation de P-1-(1,2,3-triazolyl)--Esoc-GILKEPVQGA-SEAoff est illustrée sur le schéma 4 ci-dessous.

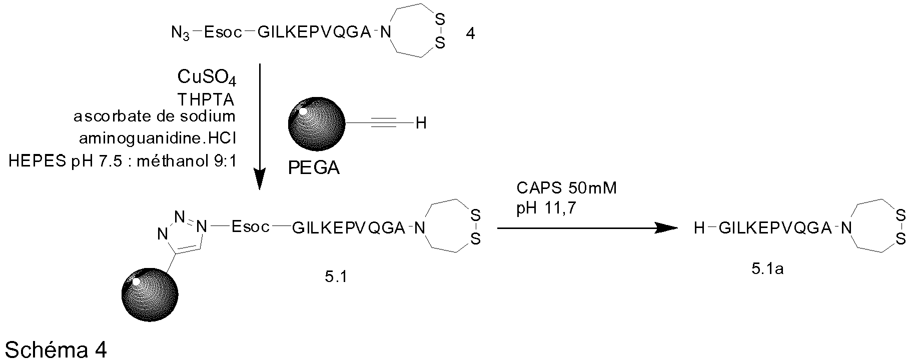

### Mode opératoire :

La résine Aminométhyle PEGA 800 (0.4 mmol/g) est couplée avec l'acide pentynoïque selon la procédure décrite dans WO2011/058188. La résine alcyne résultante (2 eq) est ajoutée à une solution de l'azidopeptide **4** (10 µmol) dans 1.67 ml un mélange 9 :1 de tampon HEPES 100mM pH = 7.5 et de méthanol. La suspension résultante est déoxygénée par bullage continu d'argon (30 min), puis le chlorhydrate d'aminoguanidine (1.1 mg, 1 eq), la tris(hydroxypropyl)triazolylméthyl-amine (THPTA) (260 mg, 60 eq), le sulfate de cuivre pentahydrate (75 mg, 30 eq) et l'ascorbate de sodium (119 mg, 60 eq) sont ajoutés et la suspension agitée par bullage d'argon pendant 30 min à TA. La peptidyl résine **5.1** est ensuite lavée abondamment avec successivement: de l'eau (dé-ionisée), une solution d'EDTA 250 mM pH 4.2, de l'eau, du méthanol, du DMF et enfin de l'eau.

Le greffage est contrôlé en traitant une petite quantité de peptidyl résine par une solution 50 mM de CAPS, dont le pH a été ajusté à 11.7 à l'aide de soude (2 x 30 min), puis acidification à pH = 3 à l'aide de TFA.

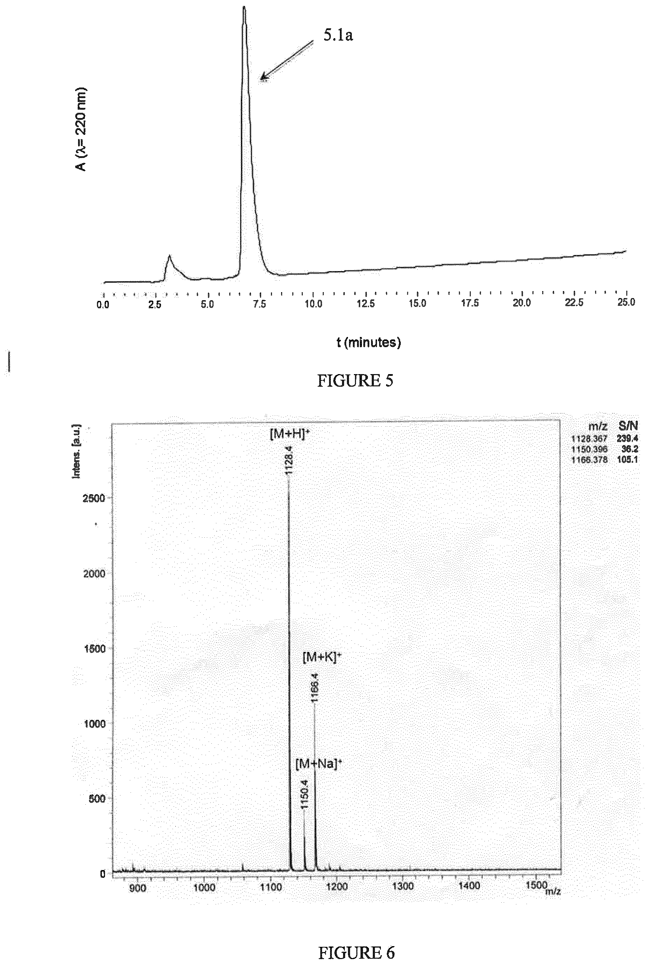

Analyse HPLC et MS de **5.1a** :
HPLC : Tampon A : H₂O/TFA (1:0.1% v/v), Tampon B : CH₃CN/TFA (1:0.1% v/v/v). Résultat en figure 5. RP-HPLC sur une colonne C18 Nucléosil (300 Å, 5 µm, 4.6 x 250 mm) en utilisant un gradient linéaire 25-45% B en 20 min, débit 1 mL/min, détection DAD. Résultat en figure 6.

### I-C- Transformation de la résine 5.1 en résine thioester 6.1

Une solution de tris(2-carboxyethyl)phosphine (TCEP) à 300 mmol/L est préparée dans un tampon phosphate 0.2 M à pH=7.3. De l'acide 3-mercaptopropionique (MPA) (10% en vol, 0.1 mL, 1.14 mmol) est alors additionné. Le pH de la solution obtenue est ajusté à pH=4.

La solution précédente (V=200 µL) est introduite sur la résine **5.1** (4,5 µmol) conditionnée dans une seringue équipée d'un fritté. Le milieu réactionnel est agité 24 h à T= 37°C. La résine est lavée (3x 300 µL x 1 min) par une solution d'acide 3-mercaptophénylacétique (MPAA, 500 mmol/L) préparée dans un tampon phosphate de sodium 0.1 M à pH=7.2.

### I-D- Ligation du peptide 7.1 H-C(StBu)HHLEPGG-SEAoff. Synthèse de la peptidyl résine 5.2. Cycle 1

L'expérience est réalisée en boite à gant ([O₂] <50 ppm). Le peptide **7.1** (9.4 mg, 0.0068 mmol, 1.5 eq) est dissous dans la solution précédente de MPAA (270 µL 500 mmol/L, tampon phosphate pH=7.2) puis transféré sur la peptidyl résine **6.1.** Le milieu réactionnel est agité 24 h à 37°C. Apres réaction, la résine est drainée puis lavée par du tampon A (H₂O/TFA)(1/0.05% v/v, 3x 300 µL x 2 min).

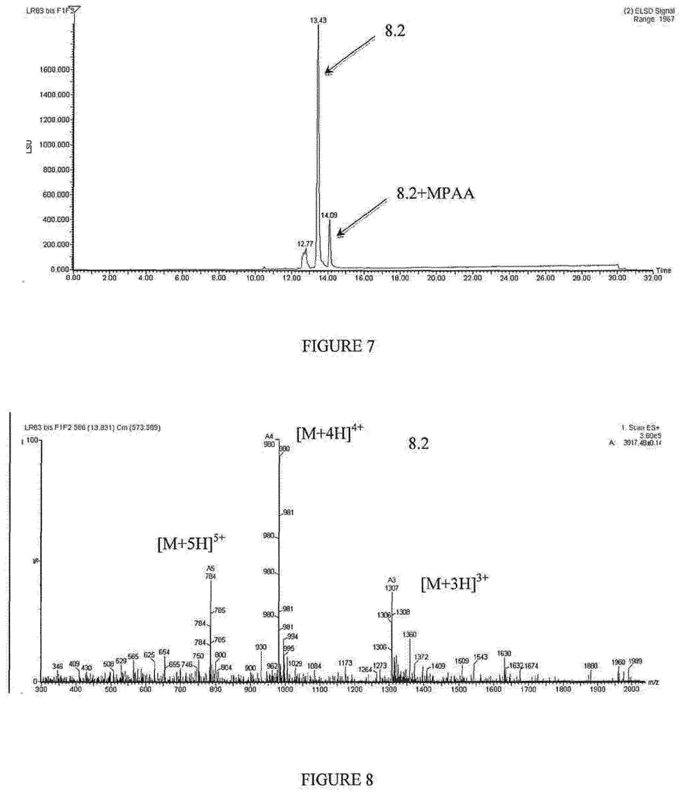

Analyse LC-MS : Chromatogramme RP-HPLC de **8.2.** Voir figure 7 : le pic **8.2** le plus important représente le produit **8.2** isolé sous forme de dimère (disulfure). Ce dimère se forme lors de la coupure du produit en milieu basique, par oxidation à l'air.

LC-MS : Tampon A : H₂O/TFA (1/0.05% v/v), Tampon B : CH₃CN/H₂O/TFA (4/1/0.05% v/v/v). RP-HPLC sur une colonne C18 Xbridge BEH (300 Å, 3.5 µm, 4.6 x 150 mm) en utilisant un gradient linéaire 0-100% B en 30 min, débit=1 mL/min, détection ELS. MS : Mode Ionisation Electrospray positif, voltage cône 30V, analyseur quadripolaire. Voir figure 8 : les pics les plus importants représentent le produit **8.2** isolé sous forme de dimère (disulfure).

### I-E- Transformation de la peptidyl résine 5.2 en résine 6.2 :

La peptidyl résine **5.2** est transformée en résine **6.2** thioester de MPA en utilisant le protocole utilisé ci-dessus pour la transformation de **5.1** en **6.1.**

### I-F- Synthèse de la peptidyl résine 5.3. Cycle 2

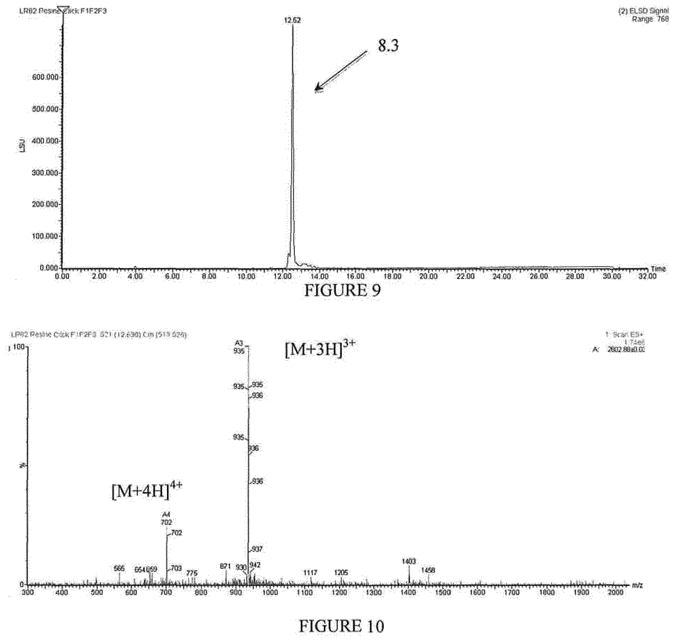

Le peptide **7.2** (X = Ala, 9.5 mg, 0.0068 mmol, 1.5 eq) est mis en réaction avec la peptidyle résine **6.2** en utilisant un protocole de synthèse strictement identique à celui utilisé pour la peptidyl résine **5.2** (cycle 1).
Analyse LC-MS : Chromatogramme RP-HPLC de **8.3** Voir figure 9.
LC-MS : Tampon A : H₂O/TFA (1/0.05% v/v), Tampon B : CH₃CN/H₂O/TFA (4/1/0.05% en v/v/v). RP-HPLC sur une colonne C18 Xbridge BEH (300 Å, 3.5 µm, 4.6 x 150 mm) en utilisant un gradient linéaire 0-100% B en 30 min, débit 1 mL/min, détection ELS. MS : Mode Ionisation Electrospray positif, voltage cône 30V, analyseur quadripolaire. Voir figure 10.

### I-G- Synthèse du peptide H-CILKEPVHGA-NH₂ (peptide 9)

La synthèse de ce peptide est décrite dans :
Ollivier, N.; Dheur, J.; Mhidia, R.; Blanpain, A.; Melnyk, O. Organic Letters 2010, 12, 5238-41.

### I-H- Synthèse du peptide 11a :

La réaction est réalisée dans une boite à gant ([O₂] <50 ppm) sous atmosphère d'azote. Une solution de TCEP à 300 mmol/L est préparée dans un tampon phosphate 0.2M à pH=7.3. L'acide 3-mercaptophénylacétique (MPAA 84 mg, 0.5 mmol) est alors additionné. Le pH de la solution TCEP/MPAA obtenue est ajusté à pH=6.5.

Le peptide **9** (4.6 mg, 0,0068 mmol, 1.5 eq) est dissous dans la solution précédente de TCEP/MPAA (110 µL) puis transféré sur la peptidyl résine **5.3.** Le milieu réactionnel est agité 24 h à 37°C. Après la réaction de ligation, la peptidyl résine **10** est drainée puis lavée par du tampon A (H₂O/TFA (1/0.05% v/v) (3 x 300 µL x 2 min).

La coupure de la peptidyl résine **11** est réalisée en boite à gant ([O₂] <50 ppm). La peptidyl-résine **11** est coupée par l'addition d'une solution de NaOH (500 µL, 10⁻² M), sous agitation pendant 15 min à 37°C. Les billes sont filtrées, lavées et le filtrat est lyophilisé.

Le peptide **11a** est purifié directement par chromatographie liquide haute performance en phase inverse (RP-HPLC) (Colonne C18 Nucleosil (d=1 cm, L=20 cm, 120 Å, 5 µm), détection UV (215 nm), tampon A : H₂O/TFA (1:0.05% en v/v), tampon B : CH₃CN/H₂O/TFA (4:1:0.05% en v/v/v), gradient : tampon B (0 à 20% en 5 min puis 20 à 42% en 60 min, 6 mL/min)). 4.2 mg de peptide 11 sont obtenus (R= 21%).

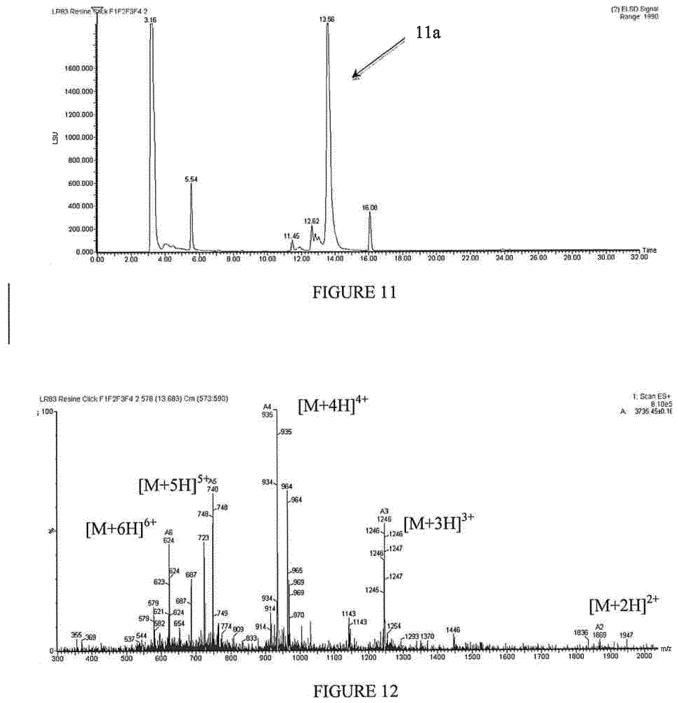

Analyse LC-MS Chromatogramme RP-HPLC du peptide **11a.** Le chromatogramme est représenté sur la figure 11.

LC-MS : Tampon A : H₂O/TFA (1/0.05% v/v), Tampon B : CH₃CN/H₂O/TFA (4/1:0.05% v/v/v). RP-HPLC sur une colonne C18 Xbridge BEH (300 Å, 3.5 µm, 4.6 x 150 mm) en utilisant un gradient linéaire 0-100% B en 30 min, débit 1 mL/min, détection ELS. MS : Mode Ionisation Electrospray positif, voltage cône 30V, analyseur quadripolaire. Le spectre de masse est représenté sur la figure 12.

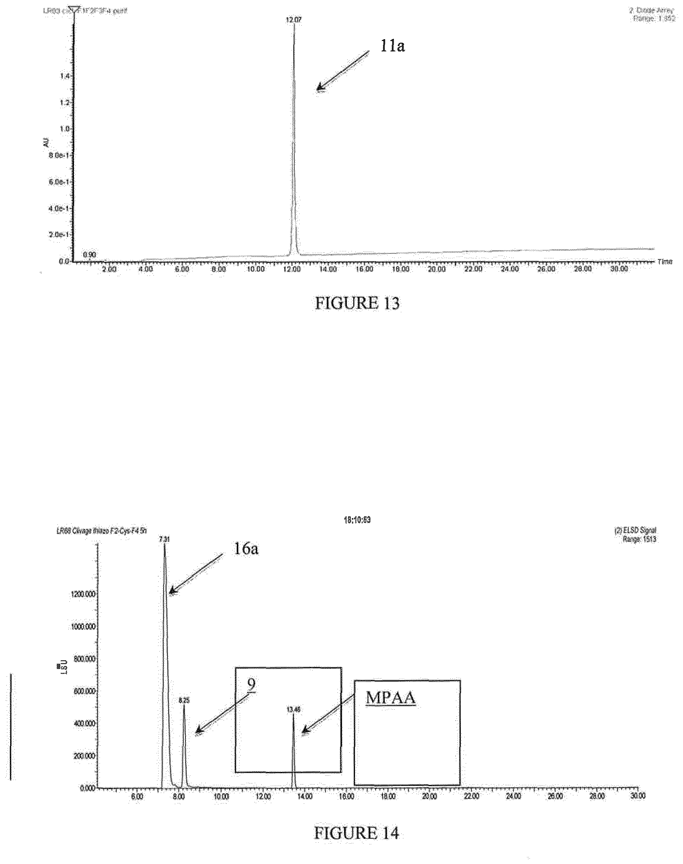

Chromatogramme RP-HPLC du peptide **11a** purifié. Le chromatogramme est représenté sur la figure 13.

### II- SYNTHESE DU PEPTIDE H-CHHLEPGG-CiLKEPVHGA-NH₂ 16a

### II-A- Synthèse de la résine 12 : Pal-ChemMatrix 5-amino-5-oxopentanoique acide

On procède suivant le schéma 5 ci-dessous :

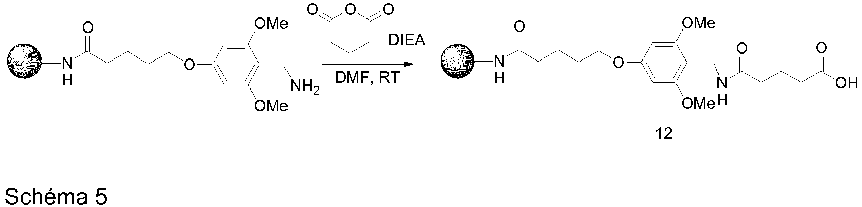

Mode opératoire : L'anhydride pentanedioique (129 mg, 1 mmol, 10 eq) est dissous dans un minimum de DMF puis transféré directement sur la resine Pal-ChemMatrix (263 mg, 0.1 mmol, δ= 0.43 mmol/g, 1 eq). De la *N*,*N*-Diisopropyléthylamine (DIEA) (197 µL, 1 mmol, 10 eq) est additionnée. Le milieu réactionnel est agité 1.5 heures sous atmosphère d'argon à température ambiante. La résine est lavée successivement au DMF (2 x 2 min), CH₂Cl₂ (2 x 2 min) puis drainée. Un test TNBS est effectué pour vérifier l'absence d'amine libre.

### II-B- Synthèse de la résine 13 : PAL-ChemMatrix N-(3-amino-2-hydroxypropyl)amide

Cette synthèse est mise en oeuvre suivant le schéma 6 ci-dessous :

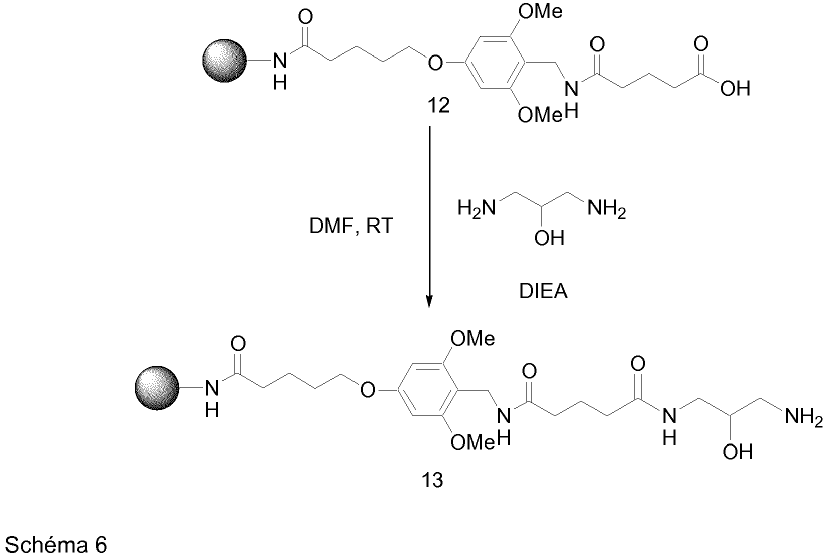

Mode opératoire : La 1,3 diamino-2-propanol (99 mg, 1 mmol, 10 eq) et le Benzotriazol-1-yloxy-tripyrrolidinophosphoniumhexafluorophosphate (PyBOP) (114 mg, 0.2 mmol, 2 eq) sont dissous dans un minium de DMF puis transférés directement sur la résine **12.** De la DIEA (191 µL, 1 mmol, 10 eq) est additionnée. Le milieu réactionnel est agité 2 heures sous atmosphère d'argon à température ambiante. La résine **13** est lavée successivement au DMF (2 x 2 min), CH₂Cl₂ (2 x 2 min) puis drainée. Un test TNBS est effectué pour vérifier la présence d'amine libre.

### II-C- Synthèse de la peptidyl résine thiazolidine 15

Cette synthèse est mise en oeuvre suivant le schéma 7 ci-dessous :

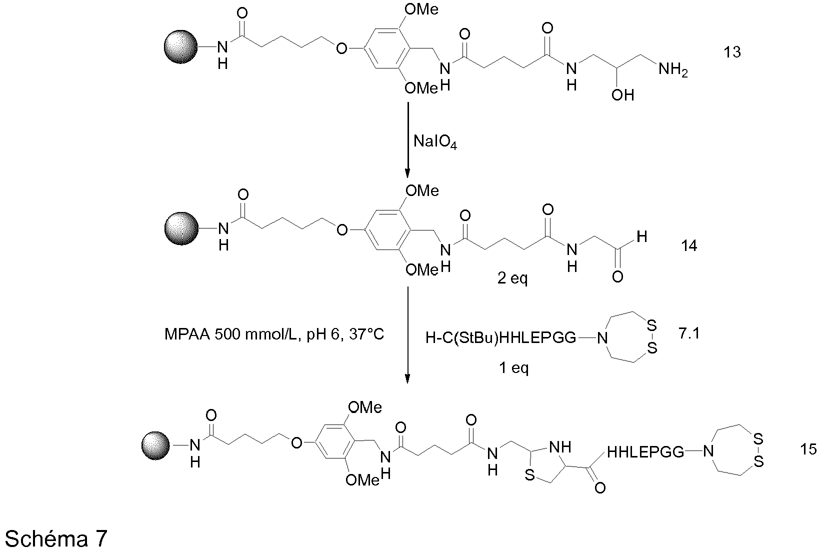

Mode opératoire : L'oxydant periodate de sodium (NaIO₄) (1.2 mg, 5.7 µmol, 4 eq) est dissous dans un minimum de tampon phosphate 0.1 M à pH=7.2 puis transféré sur la résine **13** (7.2 mg, 2.9 µmol, 2 eq). Le milieu réactionnel est agité pendant 15 minutes à température ambiante. L'excès d'oxydant est alors neutralisé par l'addition d'éthanol amine (1.4 µL, 22.9 µmol, 16 eq) sur la résine. Après agitation, la résine **14** est lavée par du tampon phosphate 0.1 M à pH=6.

La manipulation est réalisée en boite à gant ([O₂] <50 ppm). Le peptide **7.1** (2 mg, 1.4 µmol, 1 eq) est dissous dans V=72 µL d'une solution de MPAA 500 mmol/L préparée dans du tampon phosphate 0.1 M à pH=6. La solution de peptide est transférée sur la résine **14** précédente. Le milieu réactionnel est agité 24 heures à 37°C. L'aldéhyde en excès est neutralisé par V =200 µL d'une solution de méthylhydroxylamine (14 mmol/L préparée dans un tampon acetate d'ammonium à pH=4.5). Apres agitation, la résine 15 est drainée puis lavée (3* 300 mL x 2 min) par du tampon phosphate à pH=7.2.

### II-D- Ligation du peptide 9 H-CILKEPVHGA-NH₂. Synthèse de la peptidyl résine 16 :

Cette synthèse est mise en oeuvre suivant le schéma 8 ci-dessous :

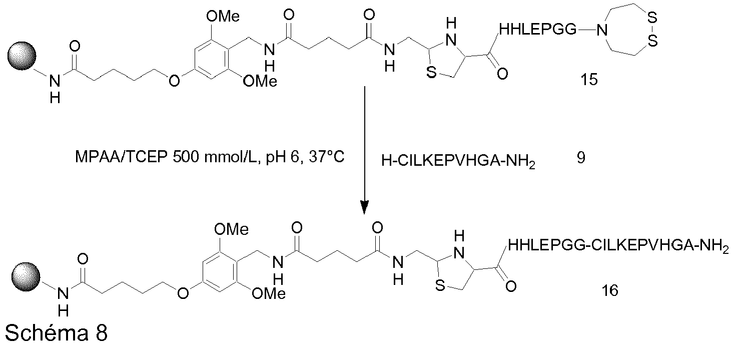

Mode opératoire : La manipulation est réalisée en boite à gant ([O₂] <50 ppm). Une solution de TCEP à 300 mmol/L est préparée dans du tampon phosphate 0.2 M à pH=7.2. De l'acide 3-mercaptophénylacétique (MPAA) (84 mg, 0.5 mmol) est alors additionné. Le pH de la solution TCEP/MPAA obtenue est ajusté à pH=6.5.

Le peptide **9** (3 mg, 2.1 µmol, 1.5 eq) est dissous dans V=70 µL de la solution précédente de TCEP/MPAA puis transféré sur la peptidyl-résine **15.** Le milieu réactionnel est agité 24 h à T=37°C. Après réaction de ligation, la résine **16** est drainée puis lavée par du tampon A : H₂O/TFA (1:0.05% en v/v) (3 x 300 µL x 2 min).

### II-E- Coupure de la peptidyl résine 16, synthèse du peptide H-CHHLEPGG-CILKEPVHGA-NH₂ 16a

Cette synthèse est mise en oeuvre suivant le schéma ci-dessous :

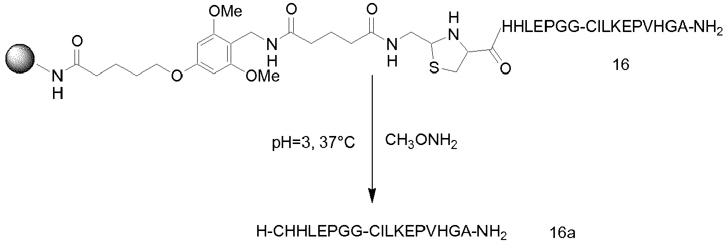

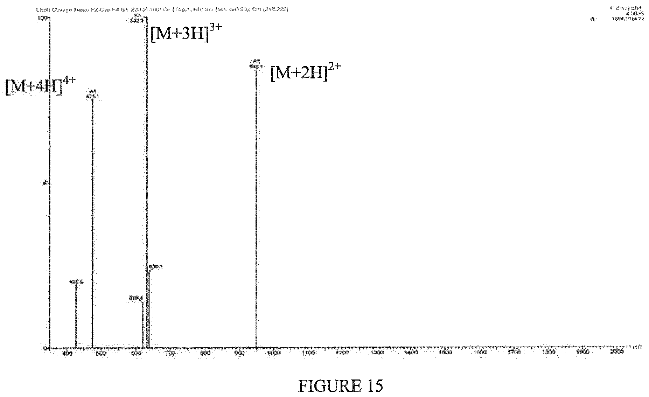

Mode opératoire : La manipulation est réalisée en boite à gant ([O₂] <50 ppm). La peptidyl résine **16** est coupée par l'addition de V= 500 µL d'une solution de méthylhydroxylamine (300 mmol/L à pH=3 préparée dans du tampon phosphate) sous agitation pendant 3 heures à 37°C. Les billes sont filtrées, lavées et le filtrat est lyophilisé.
Analyse LC-MS Chromatogramme RP-HPLC du peptide **16a** H-CHHLEPGG-CILKEPVHGA-NH₂
LC-MS : Tampon A : H₂O/TFA (1:0.05% en v/v), Tampon B : CH₃CN/H₂O/TFA (4:1:0.05% en v/v/v). RP-HPLC sur une colonne C18 Xbridge BEH (300 Å, 3.5 µm, 4.6 x150 mm) en utilisant un gradient linéaire A/B : 0-100% B en 30 min, débit=1 mL/min, detection ELS. MS : Mode Ionisation Electrospray positif, voltage cône 30V, analyseur quadripolaire. La figure 14 illustre les spectres RP-HPLC obtenus : Le pic marqué RsMPAA représente les résidus de MPAA. Sur la figure 15 on observe le spectre de masse.

### III- Synthèse du peptide H-AAAAAKDYIRN- CIIGKGRSYKGTVSITKSGIK-CQPWSSMIPHEHSFLPSSYRGKDLQENY-CRNPRGEEGGPWCFTSNPEVRYEVCDIPQCSEVK(biotine)-NH₂ 29

### III-A- Synthèse du fragment N₃-ESOC-AAAAAKDYIRN-SEAoff 19

Cette synthèse est mise en oeuvre suivant le schéma ci-dessous :

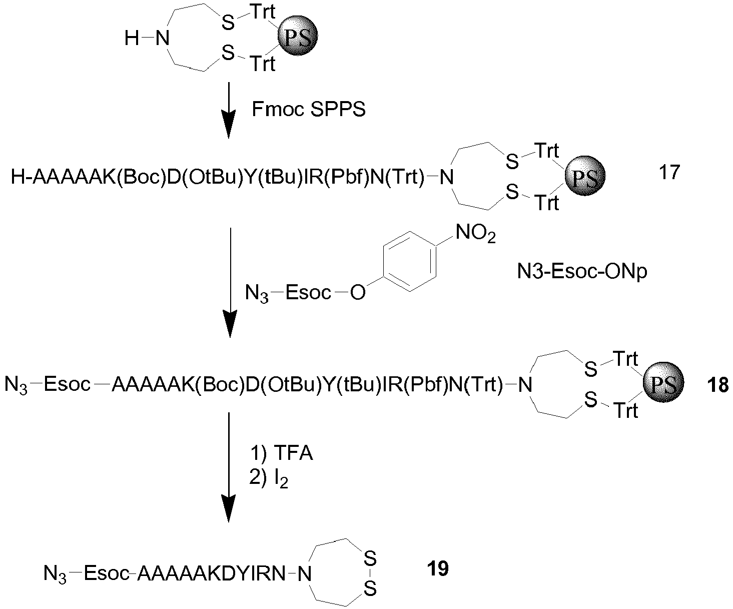

### - Synthèse de la peptidyl résine 17 H-AAAAAK(Boc)D(OtBu)Y(tBu)IR(Pbf)N(Trt)-SEA-PS (PS = polystyrène)

Cette peptidyl résine est synthétisée à une échelle de 0.25 mmole en suivant le protocole décrit dans Ollivier, N.; Dheur, J.; Mhidia, R.; Blanpain, A.; Melnyk, O. Organic letters 2010, 12, 5238-41.

Après synthèse, la résine est lavée successivement avec du CH₂Cl₂ (2 x 2 min) puis du DMF (2 x 2 min) et est engagée dans l'étape suivante.

### - Synthèse du fragment N₃-ESOC-AAAAAKDYIRN-SEAoff 19

Le bras activé N₃-Esoc-ONp est synthétisé selon la procédure décrite dans WO2011/058188. Ce carbonate mixte de 2-[2-(2-azido-éthoxy)-éthylsulfonyle]-éthyle et de 4-nitrophényle (145 mg, 0.4 mmol, 1.5 eq) est dissous dans un minimum de DMF puis transféré directement sur la résine **17.** De la *N*-méthylmorpholine (55 µL, 0,5 mmol, 2 eq) est additionnée. Le milieu réactionnel est agité 12 heures sous atmosphère d'argon à température ambiante. La résine **18** est lavée successivement au DMF (2 x 2 min), CH₂Cl₂ (2 x 2 min) et Et₂O (2 x 2 min) puis séchée sous vide.

La déprotection finale des chaînes latérales et le décrochage du peptide de la résine sont réalisés par l'action d'un mélange TFA/TIS/DMSO/H₂O (20 mL, 92.5/2.5/2.5/2.5% en v/v) pendant 1h30. Le peptide est précipité dans un mélange froid d'éther diéthylique/heptane (200 mL, 1:1 v/v), centrifugé, puis dissous dans un minimum d'eau et lyophilisé.

Le peptide **19** brut (100 mg, 0.05 mmol) est dissous dans du tampon phosphate de sodium (26 mL, 0.2 M, pH=7.2) puis oxydé en présence de *N*,*N*,*N'*,*N'*-tétraméthyiazodicarboxamide (TMAD) (18.4 mg, 0.1 mmol, 2 eq) pendant 20 minutes. Le milieu réactionnel est purifié directement par chromatographie liquide haute performance en phase inverse (RP-HPLC) (Colonne C18 Nucleosil (d=1 cm, L=20 cm, 120 Å, 5 µm), détection UV (λ=215 nm), tampon A : H₂O/TFA (1:0.05% en v/v), tampon B : CH₃CN/H₂O/TFA (4:1:0.05% en v/v/v), gradient : tampon B (0 à 20% en 5 min puis 20 à 42% en 60 min, 6 mL/min)). 23 mg de peptide **19** sont obtenus (R= 23%).
Analyse LC-MS : Chromatogramme RP-HPLC de **19**
LC-MS : Tampon A : H₂O/TFA (1/0.05% v/v), Tampon B : CH₃CN/H₂O/TFA (4/1/0.05% en v/v/v). RP-HPLC sur une colonne C18 Xbridge BEH (300 Å, 3.5 µm, 4.6 x 150 mm) en utilisant un gradient linéaire 0-100% B en 30 min, débit 1 mL/min, détection ELS. MS : Mode Ionisation Electrospray positif, voltage cône 30V, analyseur quadripolaire.

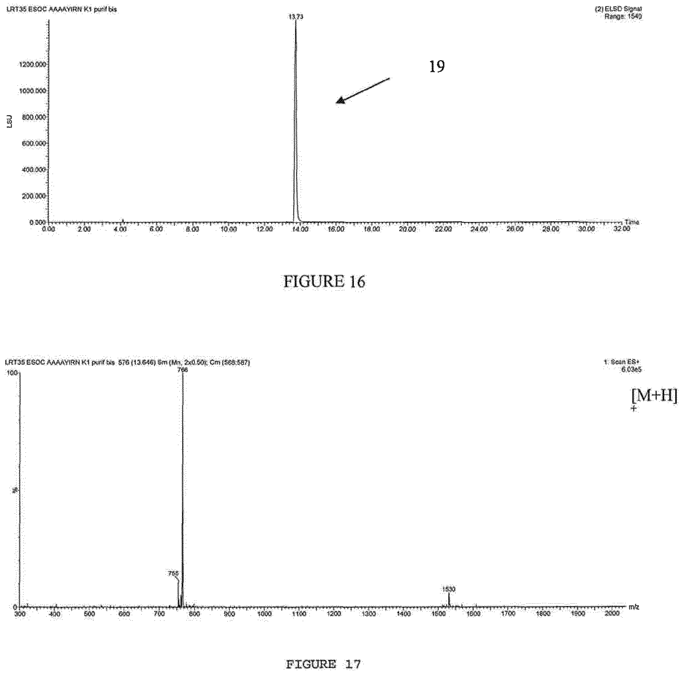

La figure 16 illustre les spectres RP-HPLC obtenus. Sur la figure 17 on observe le spectre de masse.

### III-B- Synthèse du peptide 29

Cette synthèse est mise en oeuvre suivant le schéma ci-dessous :

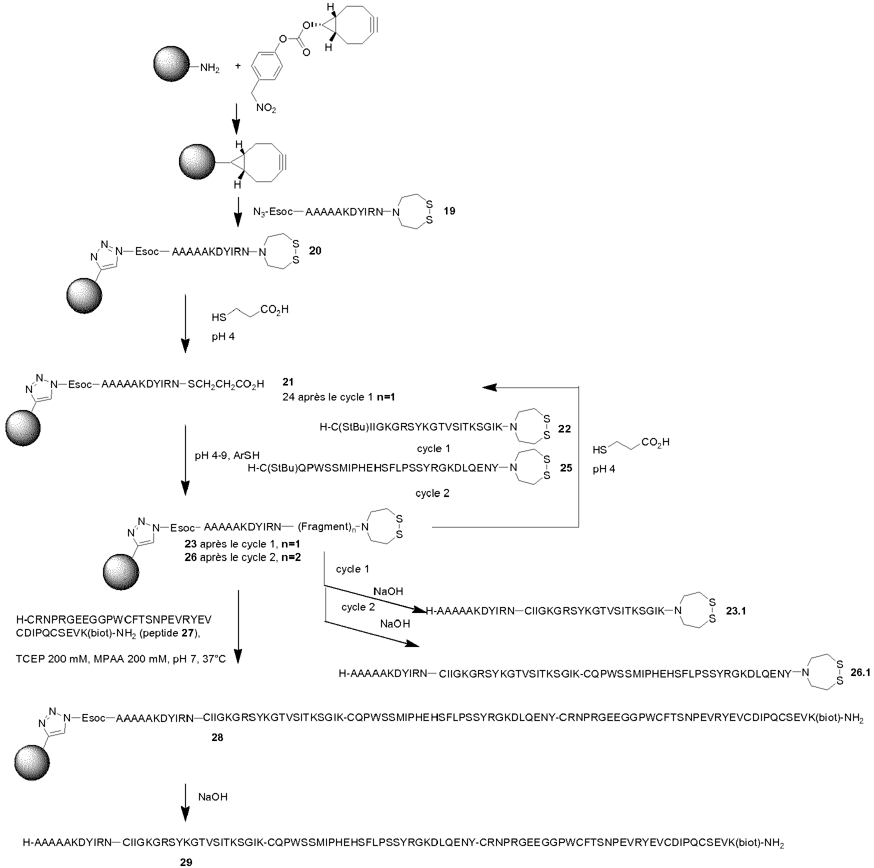

### - Greffage du peptide 19 sur un support solide cyclooctyne. Préparation du support 20 :

Le carbonate mixte de bicyclo[6.1.0]non-4-yn-9-ylmethyle et de 4-nitrophenyl) (176mg, 0.56 µmol, 3 eq), préparé selon la procédure décrite dans Dommerholt, J. et al., Angewandte Chem., Int. Ed. 2010, 49, 9422-9425, est dissout dans le minimum de DMF puis transféré directement sur la résine aminométhyle PEGA¹⁹⁰⁰ (0.4 mmol/g). De la *N,N-*diisopropyléthylamine (370 µL, 3.36 mmol, 6 eq) est additionnée. Le milieu réactionnel est agité 16 heures sous atmosphère d'argon à température ambiante. La résine est lavée successivement au DMF (2 x 2 min), CH₂Cl₂ (2 x 2 min), 20% de piperidine dans du DMF puis le mélange MeOH/DMF/AcOH (9 :9 :2) (5 x 2 min), et MeOH (2 x 2 min).

La résine alcyne résultante (2 eq) est ajoutée à une solution de l'azidopeptide **19** (8.2 µmol) dans 2.4 ml un mélange 8:2 d'eau (dé-ionisée) et d'acétonitrile avec 0,1 % de TFA. La suspension est agitée à température ambiante pendant 80 heures. La peptidyl résine **20** est ensuite lavée abondamment avec successivement: de l'acétonitrile avec 0.1 % de TFA, de l'eau avec 0.1 % de TFA, de l'eau, du MeOH, du DMF, du MeOH, et enfin de l'eau.

### - Caractérisation de la peptidyl résine 20. Décrochage du peptide et formation du peptide 21 en solution (H-AAAAAKDYIRN-SEAoff 21)

20% de piperidine dans du DMF, du MeOH/DMF/AcOH (9 :9 :2), du DMF, du MeOH, et enfin de l'eau.
Analyse HPLC : Chromatogramme RP-HPLC de 21
Tampon A : H₂O/TFA (1:0.1% v/v), Tampon B : CH₃CN/TFA (1:0.1% v/v/v). RP-HPLC sur une colonne C18 nucleosil (300 Å, 5 µm, 4.6 x 250
mm) en utilisant un gradient linéaire 25-45% B en 20 min, débit 1 mL/min,
détection DAD.

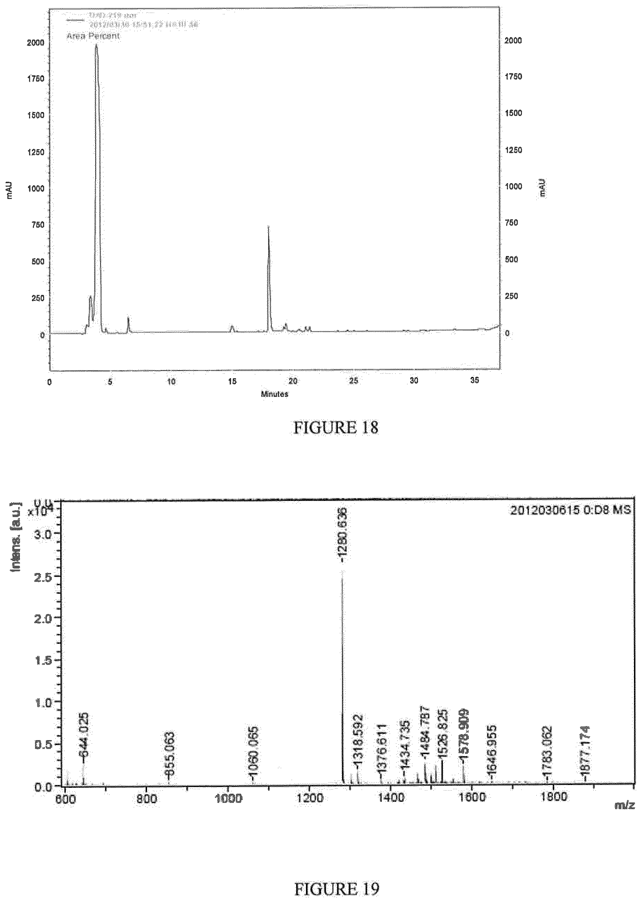

La figure 18 illustre les spectres RP-HPLC obtenus. Sur la figure 19 on observe le spectre de masse du produit **21.**

### - Synthèse du peptide 22 H-C(StBu)IIGKGRSYKGTVSITKSGIK-SEAoff

Ce peptide est synthétisé à une échelle de 0.25 mmole en suivant le protocole décrit dans Ollivier, N.; Dheur, J.; Mhidia, R.; Blanpain, A.; Melnyk, O. Organic letters 2010, 12, 5238-41.

### - Synthese du peptide 25 H-C(StBu)QPWSSMIPHEHSFLPSSYRGKDLQENY-SEAoff

Ce peptide est synthétisé à une échelle de 0.25 mmole en suivant le protocole décrit dans Ollivier, N.; Dheur, J.; Mhidia, R.; Blanpain, A.; Melnyk, O. Organic letters 2010, 12, 5238-41.

### - Synthese du peptide 27

H-CRNPRGEEGGPWCFTSNPEVRYEVCDIPQCSEVK(biotine)-NH₂
Ce peptide est synthétisé à une échelle de 0.25 mmole en suivant le protocole décrit dans Ollivier, N.; Dheur, J.; Mhidia, R.; Blanpain, A.; Melnyk, O. Organic letters 2010, 12, 5238-41.

### Transformation de la résine 20 en résine thioester 21

Une solution de tris(2-carboxyethyl)phosphine (TCEP) à 200 mmol/L est préparée dans un tampon phosphate 0.2 M en présence de guanidine (6M) à pH=7.2. De l'acide 3 mercaptopropionique (MPA) (5% en vol, 0.05 mL, 0.58 mmol) est alors additionné. Le pH de la solution obtenue est ajusté à pH=4.

La solution précédente (V=150 µL) est introduite sur la résine **20** (0.5 µmol) conditionnée dans une seringue équipée d'un fritté. Le milieu réactionnel est agité 24 h à T= 37°C. La résine est lavée (3x 150 µL x 1 min) par une solution de d'acide 3-mercaptophénylacétique (MPAA, 300 mmol/L) préparée dans un tampon phosphate de sodium 0.1 M en présence de guanidine (6M) à pH=7.2.

### - Ligation du peptide 22 H-C(StBu)IIGKGRSYKGTVSITKSGIK-SEAoff. Synthèse de la peptidyl résine 23

L'expérience est réalisée en boite à gant ([O₂] <50 ppm). Le peptide **22** (2.3 mg, 0.75 µmol, 1.5 eq) est dissous dans la solution précédente de MPAA (150 µL, 300 mmol/L, tampon phosphate 0.1M en présence de guanidine (6M) à pH 7.2) puis transféré sur la peptidyl résine **21.** Le milieu réactionnel est agité 24 h à 37°C. Après réaction, la résine est drainée puis lavée par du tampon phosphate de sodium 0.2 M en présence de guanidine (6M) à pH=7.2 (3x 150 µL x 2 min).

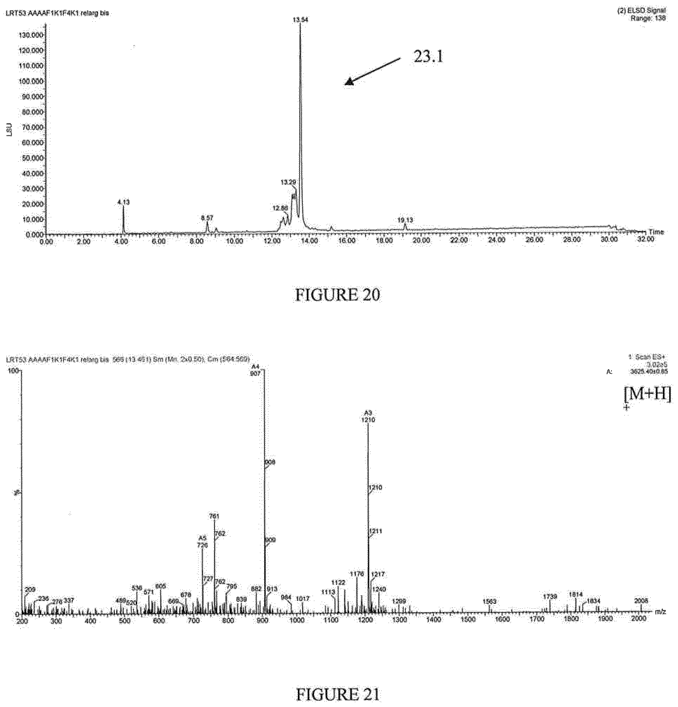

Analyse LC-MS : Chromatogramme RP-HPLC de **23.**1 obtenu après décrochage du peptide en milieu basique à partir de la peptidyl résine **23** (figure 20).

LC-MS : Tampon A : H₂O/TFA (1/0.05% v/v), Tampon B : CH₃CN/H₂O/TFA (4/1/0.05% v/v/v). RP-HPLC sur une colonne C18 Xbridge BEH (300 Å, 3.5 µm, 4.6 x 150 mm) en utilisant un gradient linéaire 0-100% B en 30 min, débit=1 mL/min, détection ELS. MS : Mode Ionisation Electrospray positif, voltage cône 30V, analyseur quadripolaire (figure 21).

La peptidyl résine **23** est transformée en résine **24** thioester de MPA en utilisant le protocole utilisé ci-dessus pour la transformation de **20** en **21.**

### - Ligation du peptide 25 H-C(StBu)QPWSSMIPHEHSFLPSSYRGKDLQENY-SEAoff. Synthèse de la peptidyl résine 26

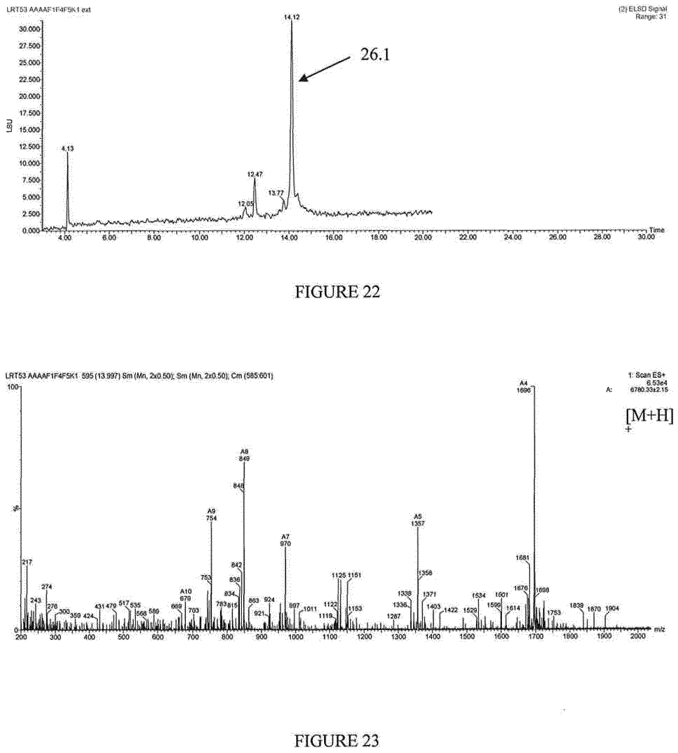

Le peptide **25** (3 mg, 0.75 µmol, 1.5 eq) est mis en réaction avec la peptidyle résine **24** en utilisant un protocole de synthèse strictement identique à celui utilisé pour la synthèse de la résine peptidyl **23** (cycle 1).
Analyse LC-MS : Chromatogramme RP-HPLC du peptide **26.1** obtenu après décrochage du peptide en milieu basique à partir de la peptidyl résine **26** (figure 22).

LC-MS : Tampon A : H₂O/TFA (1/0.05% v/v), Tampon B : CH₃CN/H₂O/TFA (4/1/0.05% en v/v/v). RP-HPLC sur une colonne C18 Xbridge BEH (300 Å, 3.5 µm, 4.6 x 150 mm) en utilisant un gradient linéaire 0-100% B en 30 min, débit 1 mL/min, détection ELS. MS : Mode Ionisation Electrospray positif, voltage cône 30V, analyseur quadripolaire (figure 23).

### - Synthèse du peptide 29

La réaction est réalisée dans une boite à gant ([O₂] <50 ppm) sous atmosphère d'azote. Une solution de TCEP à 200 mmol/L est préparée dans un tampon phosphate 0.2 M en présence de guanidine (6M) à pH=7.2. L'acide 3-mercaptophénylacétique (MPAA 33 mg, 0.2 mmol) est alors additionné. Le pH de la solution TCEP/MPAA obtenue est ajusté à pH=6.5.

Le peptide **27** (3.4 mg, 0.75 µmol, 1,5 eq) est dissous dans la solution précédente de TCEP/MPAA (150 µL) puis transféré sur la peptidyl résine **26.** Le milieu réactionnel est agité 24 h à 37°C. Après la réaction de ligation, la peptidyl résine **28** est drainée puis lavée par du tampon phosphate de sodium 0.2 M en présence de guanidine (6M) pH=7.2.

### - Coupure de la peptidyl résine 28, synthèse du peptide 29

La manipulation est réalisée en boite à gant ([O₂] <50 ppm). La peptidyl-résine **28** est coupée par l'addition d'une solution de NaOH (500 µL, 10⁻² M), sous agitation pendant 5 min à 37°C. Les billes sont filtrées, lavées et le filtrat est lyophilisé.

### Analyse LC-MS

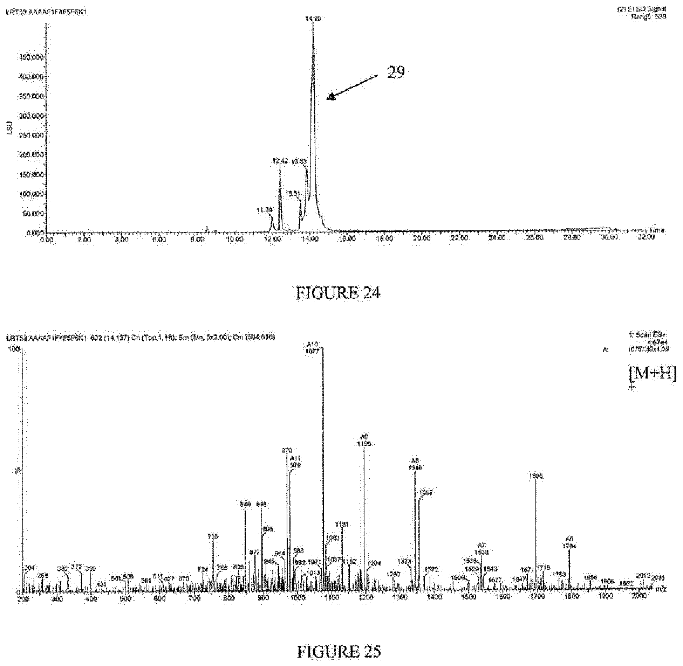

Chromatogramme RP-HPLC du peptide **29** (figure 24)
LC-MS : Tampon A : H₂O/TFA (1/0.05% v/v), Tampon B : CH₃CN/H₂O/TFA (4/1:0.05% v/v/v). RP-HPLC sur une colonne C18 Xbridge BEH (300 Å, 3.5 µm, 4.6 x 150 mm) en utilisant un gradient linéaire 0-100% B en 30 min, débit 1 mL/min, détection ELS. MS : Mode Ionisation Electrospray positif, voltage cône 30V, analyseur quadripolaire (figure 25).

## Revendications

1. Procédé d'assemblage de fragments peptidiques pour fabriquer un polypeptide comportant n fragments peptidiques et au moins n-1 acides aminés portant une fonction thiol, représenté par la formule :
A₁-C₁-A₂-C₂-A₃-...-Cᵢ₋₁-Aᵢ-....-Cₙ₋₁-Aₙ (I)
dans laquelle A₁, A₂, A₃, ... Aᵢ ..., Aₙ sont des fragments peptidiques,
C₁, C₂, C₃ ... Cᵢ₋₁ ... Cₙ₋₁ sont des restes d'acides aminés portant une fonction thiol,
n est compris entre 3 et 50,
et
i est un nombre entier quelconque compris entre 2 et n, ce procédé faisant intervenir :
(a1) au moins une étape de préparation d'un fragment Y-A₁-SEAoff (II₁) dans lequel A₁ représente un fragment peptidique dont le C-terminal porte un groupement bis(2-sulfanyléthyl)amino cyclique 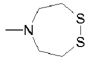 appelé SEAoff, et
Y est un fragment capable de réagir avec une fonction d'un support solide de façon à former une liaison entre A₁ et un support solide,
(b) au moins une étape de réaction de Y-A₁-SEAoff (II₁) avec un support solide noté □-Y', □ représentant le support solide lui-même et Y' représentant une fonction réactive capable de réagir avec Y pour former un groupement Z suivant le schéma : 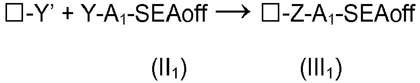
(a2) au moins une étape de préparation d'un fragment H-C₁(PG₁)-A₂-SEAoff (II₂) dans laquelle C₁, A₂ et SEAoff sont définis comme ci-dessus et (PG₁) représente H ou un groupement protecteur du thiol de l'amino-acide C₁,
(c1) au moins une étape de préparation d'un peptide-thioester de formule (III₁') à partir du bis(2-sulfanyléthyl)amino peptide □-Z-A₁-SEAoff (III₁) suivant le schéma : 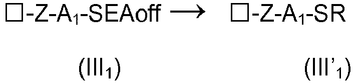 par action d'un thiol R-SH, éventuellement en présence d'un agent réducteur des disulfures cycliques, R pouvant être un radical alkyle ou aryle éventuellement substitué,
(d1) au moins une étape de condensation de (III'ᵢ) sur le fragment peptidique (II₂) en présence d'un thiol aromatique ArSH dans des conditions où PG₁ est éliminé lorsqu'il est différent de H: 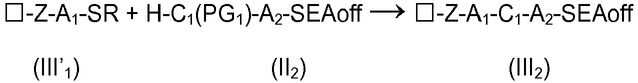
(an-1) au moins une étape de préparation d'un fragment Cₙ₋₁(PG ₙ₋₁)-An où (PGₙ₋₁) représente H ou un groupement protecteur du thiol de l'amino-acide Cₙ₋₁,
(dn-1) Au moins une étape de condensation de Cₙ₋₁(PGₙ₋₁)-An avec □-Z-
A₁-C₁-A₂-...Cᵢ₋₁Aᵢ-...Cₙ₋₂Aₙ₋₁SEAoff (IIIₙ₋₁)
en présence d'un thiol aromatique ArSH dans des conditions où PGₙ₋₁ est éliminé lorsqu'il est différent de H pour donner :
□-Z-A₁-C₁-A₂-...Cᵢ₋₁Aᵢ-...Cₙ₋₁Aₙ (IVₙ),
ledit procédé comportant en outre, pour tout i = 2,...n-2,
(ci) au moins une étape de transformation de
□-Z-A₁-C₁-...Cᵢ₋₁AᵢSEAoff (IIIᵢ)
en
□-Z-A₁-C₁-...Cᵢ₋₁-AᵢSR (III'ᵢ)
par action d'un thiol R-SH, éventuellement en présence d'un agent réducteur de disulfures cycliques,
(di) au moins une étape de condensation de Cᵢ(PGᵢ)Aᵢ₊₁SEAoff où (PGᵢ) représente H ou un groupement protecteur du thiol de l'amino-acide Cᵢ, en présence d'un thiol aromatique ArSH dans des conditions où PGi, lorsqu'il est différent de H, est éliminé, avec
□-Z-A₁-C₁-...Cᵢ₋₁Aᵢ-SR (III'ᵢ)
pour donner
□-Z-A₁-C₁-...CᵢAᵢ₊₁SEAoff (II1ᵢ₊₁)

2. Procédé selon la revendication 1, qui comporte encore :
(e) une étape de décrochage du peptide A₁-C₁-A₂-...Cᵢ₋₁Aᵢ-...Cₙ₋₁Aₙ (I) du support solide.

3. Procédé selon la revendication 1 ou la revendication 2, dans lequel le support solide □ est choisi parmi les résines, en particulier parmi les résines à base de polystyrène, polyacrylamide, polyéthylèneglycol, cellulose, polyéthylène, polyester, latex, polyamide, polydiméthylacrylamide, les polymères hydrophiles synthétiques ou naturels,les billes de verre , les gels de silice.

4. Procédé selon l'une quelconque des revendications 1 à 3, dans lequel C₁,...Cᵢ...Cₙ sont des cystéines.

5. Procédé selon l'une quelconque des revendications 1 à 4, dans lequel PG₁,... PGᵢ... PGₙ sont des des groupements terbutyle sulfényle.

6. Procédé selon l'une quelconque des revendications 1 à 5, dans lequel :
Y' comporte une fonction choisie parmi un azoture et Y est choisi parmi les groupements comportant une fonction alcyne, ou
Y' comporte une fonction alcyne et Y est choisi parmi les groupements comportant une fonction azoture ou
Y' comporte une fonction aldéhyde, Y est H et l'acide aminé N-terminal de A₁ est choisi parmi une cystéine une sérine ou une thréonine, ou
Y' comporte une fonction aldéhyde, Y comporte un groupement NH₂ capable de former une base de Schiff.

7. Procédé selon l'une quelconque des revendications 1 à 6, dans lequel R est choisi parmi un radical alkyle comprenant de 1 à 12 atomes de carbone, linéaire ou ramifié, éventuellement substitué, ou aryle en C6-C12, éventuellement substitué.

8. Procédé selon l'une quelconque des revendications 1 à 7, dans lequel, pour tout i = 2,...n-2 , les étapes di) du procédé sont mises en oeuvre dans des conditions ou PGᵢ est enlevé sélectivement *in situ,* sans affecter SEAoff et le solvant de la réaction est un tampon aqueux, de pH compris entre 4 et 9, contenant un thiol aromatique.

9. Procédé de fabrication d'un médicament comprenant au moins :
- la fabrication d'au moins un polypeptide selon le procédé selon l'une quelconque des revendications 1 à 8, et
- son association avec un support pharmaceutiquement acceptable.

10. Support solide greffé par un peptide et répondant à la formule (III₁) ci-dessous :
□-Z-A₁-SEAoff (III₁)
dans laquelle A₁ représente un fragment peptidique, dont le C-terminal porte un groupement bis(2-sulfanyléthyl)amino cyclique 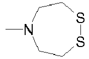 appelé SEAoff, □ représente un support solide et Z représente un groupement de liaison entre A₁ et □.

11. Kit de synthèse de polypeptides comprenant au moins un support doté d'au moins une zone de réception sur ou dans laquelle est placé au moins un support solide greffé selon la revendication 10.

## Patentansprüche

1. Verfahren zum Zusammenstellen von Peptidfragmenten, um ein Polypeptid herzustellen, das n Peptidfragmente und mindestens n-1 Aminosäuren mit Thiolanteil umfasst und der nachfolgenden Formel entspricht:
A₁-C₁-A₂-C₂-A₃-...-Cᵢ₋₁-Aᵢ-....-Cₙ₋₁-Aₙ (I)
wobei A₁, A₂, A₃, ... Aⱼ,..., Aₙ Peptidfragmente sind,
C₁, C₂, C₃... Cᵢ₋₁ ... Cₙ₋₁ Aminosäurereste mit Thiolfunktion sind, n im Bereich von 3 - 50 liegt und
i eine Ganzzahl zwischen 2 und n ist, wobei das Verfahren umfasst:
(a1) mindestens einen Schritt des Herstellens eines Fragments Y-A₁SEAoff (II₁), wobei A₁ ein Peptidfragment darstellt, dessen C-Terminus eine zyklische Bis(2-Sulfanylethyl)aminogruppe (SEAoff) 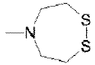 aufweist, und
Y ein Fragment ist, das mit einer Funktion eines festen Trägers derart reagieren kann, dass dadurch eine Bindung zwischen A₁ und einem festen Träger gebildet wird,
(b) mindestens einen Schritt des Umsetzens von Y-A₁SEAoff (II₁) mit einem festen Träger (□-Y', □), wobei der feste Träger sich selber darstellt und Y' eine reaktionsfähige Funktion darstellt, die mit Y eine Reaktion eingehen kann, um eine Gruppe Z nach folgenden Schema zu bilden: 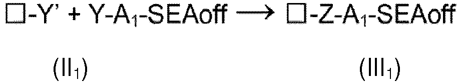
(a2) mindestens einen Schritt des Herstellens eines Fragments H-C₁(PG₁)-A₂-SEAoff (II₂), wobei C₁, A₂ und SEAoff der obigen Definition entsprechend und (PG₁) H oder eine Thiolschutzgruppe der Aminosäure C₁ darstellt,
(c1) mindestens einen Schritt des Herstellens eines Peptid-Thioesters der Formel (III₁'), ausgehend von Bis(2-sulfanylethyl)aminopeptid □-Z-A₁-SEAoff (III₁) nach folgendem Schema: 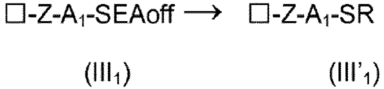 durch die Einwirkung eines Thiols R-SH, ggf. in Gegenwart eines Reduktionsmittels für zyklische Disulfide, wobei R ein Alkyl- oder ein ggf. substituiertes Arylradikal sein kann,
(d1) mindestens einen Schritt des Verdichtens von (III'₁) am Peptidfragment (II₂) in Gegenwart eines aromatischen Thiols ArSH unter Bedingungen, in denen PG₁ entfernt wird, wenn sie sich von H unterscheidet: 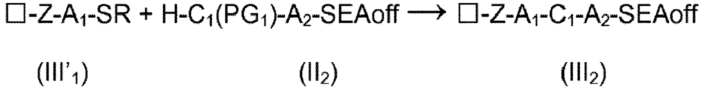
(an-1) mindestens einen Schritt des Herstellens eines Fragments Cₙ₋₁(PGₙ₋₁)-An, wobei (PGₙ₋₁) H oder eine Thiolschutzgruppe der Aminosäure Cₙ₋₁ darstellt,
(dn-1) mindestens einen Schritt des Verdichtens von Cₙ₋₁(PGₙ₋₁)-An mit
□-Z-A₁C₁-A₂-...Cᵢ₋₁Aᵢ-...Cₙ₋₂Aₙ₋1SEAoff (IIIₙ₋₁)
in Gegenwart eines aromatischen Thiols ArSH unter Bedingungen, in denen PGₙ₋₁ entfernt wird, wenn es sich von H unterscheidet, um Folgendes zu ergeben:
□-Z-A₁-C₁-A₂-...Cᵢ₋₁Aᵢ-...Cₙ₋₁Aₙ (IVₙ),
wobei das Verfahren ferner umfasst, für jedes i = 2,...n-2,
(c1) mindestens einen Schritt des Umwandelns von
□-Z-A₁-C₁-...Cᵢ₋₁AᵢSEAoff (IIIᵢ)
In
□-Z-A₁-C₁-...Cᵢ₋₁-AᵢSR (III'ᵢ)
durch die Einwirkung eines Thiols R-SH, ggf. in Gegenwart eines Reduktionsmittels für zyklische Disulfide,
(d1) mindestens einen Schritt des Verdichtens von Cᵢ(PGᵢ)Aᵢ₊₁SEAoff, wobei (PGᵢ) H oder eine Thiolschutzgruppe der Aminosäure Cᵢ darstellt, in Gegenwart eines aromatischen Thiols ArSH unter Bedingungen, in denen PGᵢ entfernt wird, wenn es sich von H unterscheidet, mit
□-Z-A₁-C₁-...Cᵢ₋₁Aᵢ-SR (III'ᵢ)
um Folgendes zu ergeben:
□-Z-A₁-C₁-...CᵢAᵢ₊₁SEAoff (IIIᵢ₊₁)

2. Verfahren nach Anspruch 1, ferner umfassend :
(e) Trennen des Peptids A1-C₁-A₂-...Cᵢ₋₁Aᵢ-...Cₙ₋₁Aₙ (I) vom festen Träger.

3. Verfahren nach Anspruch 1 oder 2, wobei der feste Träger □ aus der nachfolgenden Gruppe gewählt ist : Harze, insbesondere Harze auf Polystyrol-, Polyacrylamid-, Polyethylenglykol-, Cellulose-, Polyethylen-, Polyester., Latex-, Polyamid., Polydimethylacrylamidbasis, synthetische oder natürliche hydrophile Polymere, Glaskugeln, Kieselgel.

4. Verfahren nach einem der Ansprüche 1 - 3, wobei C₁,...Cᵢ...Cₙ Cysteine sind.

5. Verfahren nach einem der Ansprüche 1 - 4, wobei PG₁,... PGᵢ... PGₙ Tertbutylsulfenylgruppen sind.

6. Verfahren nach einem der Ansprüche 1 - 5, wobei:
Y' eine Funktion umfasst, die aus den Aziden gewählt ist, und Y aus den Gruppen gewählt ist, die eine Alkinfunktion umfassen, oder
Y' eine Alkinfunktion umfasst, und Y aus den Gruppen gewählt ist, die eine Azidfunktion umfassen, oder
Y' eine Aldehydfunktion umfasst, Y H ist und die N-terminale Aminosäure von A₁ aus Cystein, Serin oder Threonin gewählt ist, oder
Y' eine Aldehydfunktion umfasst, Y eine NH₂-Gruppe umfasst, die eine Schiff-Base bilden kann.

7. Verfahren nach einem der Ansprüche 1 - 6, wobei R aus der nachfolgenden Gruppe gewählt ist : ein lineares oder verzweigtes, ggf. substituiertes Alkylradikal mit 1 - 12 Kohlenstoffatomen oder ggf. substituiertes C6-C12-Aryl.

8. Verfahren nach einem der Ansprüche 1 - 7, wobei für jedes i = 2,...n-2 , die Schritte di) des Verfahrens unter Bedingungen ausgeführt werden, in denen PGᵢ in situ selektiv entfernt wird, ohne SEAoff zu beeinflussen, und das Lösemittel der Reaktion ein wässriger Puffer ist mit einem pH-Wert zwischen 4 und 9, der ein aromatisches Thiol enthält.

9. Verfahren zur Herstellung eines Arzneimittels, mindestens umfassend:
- Herstellen mindestens eines Polypeptids im Verfahren nach einem der Ansprüche 1 - 8 und
- Verbinden des Polypeptids mit einem pharmazeutisch verträglichen Träger.

10. Fester Träger, dem einem Peptid aufgepfropft ist und der der nachfolgenden Formel (III₁) entspricht:
□-Z-A₁-SEAoff (III₁)
wobei A₁ ein Peptidfragment darstellt, dessen C-Terminus eine zyklische Bis(2-sulfanylethyl)aminogruppe (SEAoff) 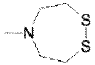 aufweist, □ einen festen Träger darstellt und Z eine Linkergruppe zwischen A₁ und □ darstellt.

11. Peptidsynthese-Set, umfassend mindestens einen Träger mit mindestens einem Aufnahmebereich, in bzw. auf dem mindestens ein verpfropfter fester Träger nach Anspruch 10 angeordnet ist.

## Claims

1. Method of assembling peptide fragments to make a polypeptide having n peptide fragments and at least n-1 amino acids bearing a thiol function, represented by the formula:
A₁-C₁-A₂-C₂-A₃-...-Cᵢ₋₁-Aᵢ-....-Cₙ₋₁-Aₙ (I)
in which
- A₁, A₂, A₃, ... Aᵢ ..., Aₙ are peptide fragments,
- C₁, C₂, C₃ ... Cᵢ₋₁ ... Cₙ₋₁ are residues of amino acids bearing a thiol function,
- n is between 3 and 50,
and
- i is any integer between 2 and n, wherein this method involves:
• (a1) at least one step for preparing a Y-A₁-SEAoff (II₁) fragment, in which A₁ represents a peptide fragment whose C-terminal bears a cyclic bis(2-sulfanylethyl)-amino called SEAoff,
and 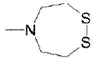 wherein Y is a fragment capable of reacting with a function of a solid support so as to form a bond between A₁ and a solid support,
• (b) at least one reaction step of Y-A₁-SEAoff (II₁) with a solid support denoted □-Y', wherein □ represents the solid support itself and Y' represents a reactive functional group capable of reacting with Y to form a group Z according to the diagram: 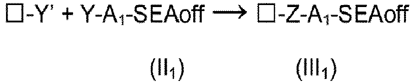
• (a2) at least one step of preparing an H-C₁(PG₁)-A₂-SEAoff (II₂) fragment in which C₁, A₂ and SEAoff are defined as above and (PG₁) represents H or a thiol protective group of the amino acid C₁,
• (c1) at least one step of preparing a peptide-thioester of formula (III₁') starting from bis(2-sulphanylethyl)amino peptide □-Z-A₁-SEAoff (III₁) according to the diagram: 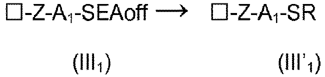 by action of an R-SH thiol, optionally in the presence of a cyclic disulfide reducing agent, wherein R is possibly an optionally substituted alkyl or aryl radical,
• (d1) at least one condensation step (III'₁) on the peptide fragment (II₂) in the presence of an ArSH aromatic thiol under conditions where PG₁ is eliminated when it is different from H: 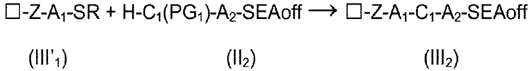
• (an-1) at least one step of preparing a Cₙ₋₁(PGₙ₋₁)-An fragment, wherein (PGₙ₋₁) represents H or a thiol protective group of the Cₙ₋₁ amino acid,
• (dn-1) at least one condensation step of Cₙ₋₁(PGₙ₋₁)-An with
□-Z-A₁-C₁-A₂-...Cᵢ₋₁Aᵢ-...Cₙ₋₂Aₙ₋₁SEAoff (IIIₙ₋₁)
in the presence of an ArSH aromatic thiol under conditions where PGₙ₋₁ is removed when it is different from H to give:
□-Z-A₁-C₁-A₂-...Cᵢ₋₁Aᵢ-...Cₙ₋₁Aₙ (IVₙ),
wherein the method further comprises for all i=2... n-2,
(ci) at least one step of transformation of
□-Z-A₁-C₁-...Cᵢ₋₁AᵢSEAoff (IIIᵢ)
in
□-Z-A₁-C₁-...Cᵢ₋₁-AᵢSR (III'ᵢ)
by the action of an R-SH thiol, optionally in the presence of a cyclic disulfide reducing agent,
• (di) at least one condensation step of Cᵢ(PGᵢ)Aᵢ₊₁ SEAoff, wherein (PGᵢ) represents H or a protective group of the thiol of the amino acid Cᵢ, in the presence of an ArSH aromatic thiol under conditions where PGᵢ, when it is different from H, is eliminated, with
□-Z-A₁₋C₁-...Cᵢ₋₁Aᵢ-SR (III'ᵢ)
to give
□-Z-A₁-C₁-...CᵢAᵢ₊₁SEAoff (IIIᵢ₊₁)

2. Method according to claim 1, which further comprises:
(e) a step of uncoupling the A₁-C₁-A₂ ... Cᵢ₋₁Aᵢ ... Cₙ₋₁Aₙ peptide (I) from the solid support.

3. Method according to claim 1 or claim 2, wherein the solid support □ is chosen from among resins, in particular from resins based on polystyrene, polyacrylamide, polyethylene glycol, cellulose, polyethylene, polyester, latex, polyamide, polydimethylacrylamide, synthetic or natural hydrophilic polymers, glass beads, silica gels.

4. Method according to any one of the claims 1 to 3, wherein C₁,... Cᵢ,... Cₙ are cysteines.

5. Method according to any one of the claims 1 to 4, wherein PG₁, ... PGᵢ ... PGₙ are terbutyl sulfenyl groups.

6. Method according to any one of the claims 1 to 5, wherein:
- Y' comprises a function chosen from an azide, while Y is chosen from among groups comprising an alkyne function, or
- Y' has an alkyne function, and Y is chosen from among groups containing an azide function or
- Y' has an aldehyde function, wherein Y is H and the amino acid N-terminal of A₁ is selected from among a cysteine, a serine or a threonine, or
- Y' contains an aldehyde function, wherein Y has an NH₂ group capable of forming a Schiff base.

7. Method according to any one of the claims 1 to 6, in which R is chosen from an alkyl radical comprising from 1 to 12 carbon atoms, linear or branched, optionally substituted, or a C6-C12 aryl, optionally substituted.

8. Method according to any one of the claims 1 to 7, wherein for all i = 2, ... n-2, the steps di) of the method are carried out under conditions where PGᵢ is selectively removed *in situ,* without affecting SEAoff and the reaction solvent is an aqueous buffer with a pH between 4 and 9 containing an aromatic thiol.

9. Method of manufacturing a medicament comprising at least:
- the manufacture of at least one polypeptide according to the process according to any one of the claims 1 to 8, and
- its association with a pharmaceutically acceptable support.

10. Solid support grafted with a peptide and having the formula (III₁) below:
□-Z-A₁-SEAoff (III₁)
in which A₁ represents a peptide fragment, whose C-terminal bears a cyclic bis(2-sulfanylethyl)amino group 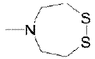 called SEAoff, wherein □ represents a solid support and Z represents a linking group between A₁ and □.

11. Polypeptide synthesis kit comprising at least one support having at least one receiving zone on which or in which is placed at least one grafted solid support according to claim 10.
